# EUROPÄISCHE PATENTSCHRIFT

(11) **EP 1 242 038 B1**
(45) Veröffentlichungstag und Bekanntmachung des Hinweises auf die Patenterteilung: **18.10.2006**
(21) Anmeldenummer: 00991201.5
(22) Anmeldetag: 15.12.2000
(51) Int. Cl.: A61K 8/49, A61K 8/41, A61Q 5/10

(54) **MITTEL ZUM FÄRBEN VON KERATINHALTIGEN FASERN**
AGENT FOR DYEING KERATIN CONTAINING FIBERS
AGENT POUR COLORER DES FIBRES KERATINIQUES

(30) Priorität: 24.12.1999 DE 19962872
(43) Veröffentlichungstag der Anmeldung: 25.09.2002
(73) Patentinhaber: Henkel Kommanditgesellschaft auf Aktien, 40589 Düsseldorf (DE)
(72) Erfinder: ROSE, David, 40723 Hilden (DE); MEINIGKE, Bernd, 51381 Leverkusen (DE); HÖFFKES, Horst, 40595 Düsseldorf (DE)
(86) Internationale Anmeldenummer: PCT/EP2000/012813
(87) Internationale Veröffentlichungsnummer: WO 2001/047475

(56) Entgegenhaltungen:
- EP-A- 0 891 971
- WO-A-01/34104
- DE-A- 2 509 152
- DE-C- 19 813 937
- DE-U- 29 909 427

## Beschreibung

Die Erfindung betrifft ein neues Oxidationsfärbemittel zum Färben von keratinhaltigen Fasern, das eine Kombination aus mindestens einem 4,5-Diaminopyrazolderivat als Entwicklerkomponente und mindestens einem halogenierten m-Aminophenol als Kupplerkomponente enthält, sowie ein Verfahren zum Färben von Keratinfasern.

Menschliches Haar wird heute in vielfältiger Weise mit haarkosmetischen Zubereitungen behandelt. Dazu gehören etwa die Reinigung der Haare mit Shampoos, die Pflege und Regeneration mit Spülungen und Kuren sowie das Bleichen, Färben und Verformen der Haare mit Färbemitteln, Tönungsmitteln, Wellmitteln und Stylingpräparaten. Dabei spielen Mittel zur Veränderung oder Nuancierung der Farbe des Kopfhaares eine herausragende Rolle. Übliche Haarfärbemittel werden, je nach gewünschter Farbe bzw. Dauerhaftigkeit der Färbung, entweder auf Basis von Oxidationsfarbstoffen oder auf Basis von direktziehenden Farbstoffen formuliert. Häufig werden auch Kombinationen von Oxidations- und direktziehenden Farbstoffen zur Erzielung spezieller Nuancen eingesetzt.

Für temporäre Färbungen werden üblicherweise Färbe- oder Tönungsmittel verwendet, die als färbende Komponente sogenannte Direktzieher enthalten. Hierbei handelt es sich um Farbstoffmoleküle, die direkt auf das Haar aufziehen und keinen oxidativen Prozeß zur Ausbildung der Farbe benötigen. Zu diesen Farbstoffen gehört beispielsweise das bereits aus dem Altertum zur Färbung von Körper und Haaren bekannte Henna. Diese Färbungen sind gegen Shampoonieren in der Regel deutlich empfindlicher als die oxidativen Färbungen, so dass dann sehr viel schneller eine vielfach unerwünschte Nuancenverschiebung oder gar eine sichtbare "Entfärbung" eintritt.

Für dauerhafte, intensive Färbungen mit entsprechenden Echtheitseigenschaften werden sogenannte Oxidationsfärbemittel verwendet. Solche Färbemittel enthalten üblicherweise Oxidationsfarbstoffvorprodukte, sogenannte Entwicklerkomponenten und Kupplerkomponenten. Die Entwicklerkomponenten bilden unter dem Einfluß von Oxidationsmitteln oder von Luftsauerstoff untereinander oder unter Kupplung mit einer oder mehreren Kupplerkomponenten die eigentlichen Farbstoffe aus. Die Oxidationsfärbemittel zeichnen sich durch hervorragende, lang anhaltende Färbeergebnisse aus.

Gute Oxidationsfarbstoffvorprodukte müssen in erster Linie folgende Voraussetzungen erfüllen. Sie müssen bei der oxidativen Kupplung die gewünschten Farbnuancen in ausreichender Intensität und Echtheit ausbilden. Sie müssen ferner ein gutes Aufziehvermögen auf die Faser besitzen, wobei insbesondere bei menschlichen Haaren keine merklichen Unterschiede zwischen strapaziertem und frisch nachgewachsenem Haar bestehen dürfen (Egalisiervermögen). Sie sollen beständig sein gegen Licht, Wärme und den Einfluß chemischer Reduktionsmittel, z. B. gegen Dauerwellflüssigkeiten. Schließlich sollen sie - falls als Haarfärbemittel zur Anwendung kommend - die Kopfhaut nicht zu sehr anfärben, und vor allem sollen sie in toxikologischer und dermatologischer Hinsicht unbedenklich sein. Es ist auch von Vorteil, wenn die Substanzen eine hohe Löslichkeit in verschiedenen Basisformulierungen besitzen. Weiterhin soll die erzielte Färbung durch Blondierung leicht wieder aus dem Haar entfernt werden können, falls sie doch nicht den individuellen Wünschen der einzelnen Person entspricht und rückgängig gemacht werden soll.

Allein mit einer Entwicklerkomponente oder einer speziellen Kuppler/Entwickler-Kombination gelingt es in der Regel nicht, eine auf dem Haar natürlich wirkende Farbnuance zu erhalten. In der Praxis werden daher üblicherweise Kombinationen verschiedener Entwickler- und Kupplerkomponenten eingesetzt; weiterhin werden in vielen Fällen direktziehende Farbstoffe zur Nuancierung verwendet. Es besteht daher ständig Bedarf an neuen, verbesserten Farbstoffkombinationen.

Es war daher die Aufgabe der vorliegenden Erfindung, neue Kombinationen von Farbstoffvorprodukten zu finden, die für den Einsatz in Oxidationsfärbemitteln geeignet sind und den an sie gestellten Anforderungen in besonderem Maße genügen.

Es wurde nun überaschenderweise gefunden, dass neue Kombinationen spezieller halogenierter m-Aminophenole als Kupplerkomponenten mit 4,5-Diaminopyrazolen als Entwicklerkomponenten die an Oxidationsfärbemittel gestellten Anforderungen in besonders hohem Maße erfüllen. So werden unter Verwendung dieser Kupplerkomponenten mit beispielsweise 4,5-Diamino-1-(2'-hydroxyethyl)pyrazol als Entwicklerkomponente brillante, sehr intensive Farbnuancen im Rot- und Magenta-Bereich erhalten, die außerordentlich licht- und waschecht sind.

Der Einsatz von 4,5-Diaminopyrazol-Derivaten als Entwicklersubstanzen in Haarfärbemitteln wird beispielsweise in der EP-B1-0 740 931 beschrieben. Die Verwendung von weiteren Derivaten ist aus den Druckschriften WO-94/08970-A1, WO-94/08969-A1 und DE 38 43 892 A 1 bekannt. Diese Schriften geben aber keine Hinweise auf die hervorragenden färberischen Eigenschaften der erfindungsgemäßen Kombinationen von 4,5-Diaminopyrazolen mit den speziellen halogenierten m-Aminophenolen.

Der Einsatz von 4-Fluor-3-aminophenol in Kombination mit Pyrazolderivaten wird in der DE-U1-299 09 427 beschrieben. Die DT-A1- 25 09 152 und die DE-C1-198 13 937 offenbaren den Einsatz von in 4-Stellung chlorierten m-Aminophenolderivaten in Haarfärbemitteln.

Die WO 01/34104 A ist ein Zwischendokument im Sinne von Artikel 54(3)(4) EPÜ und offenbart ein Haarfärbemittel enthaltend N, N'-Bis (4-aminophenyl)-1, 4-diazacycloheptan, 4,5-Diamino-1-hydroxyethylpyrazol und 4-Chlor-6-methyl-3-aminophenol

Ein erster Gegenstand der vorliegenden Erfindung ist daher ein Oxidationsfarbemittel zum Färben von keratinhaltigen Fasern, das eine Kombination enthält aus mindestens Diaminopyrazolderivat der allgemeinen Formel (II) oder einem seiner physiologisch verträglichen Salze, 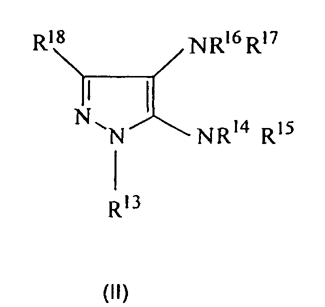 wobei R¹³, R¹⁴, R¹⁵, R¹⁶ und R¹⁷ unabhängig voneinander stehen für Wasserstoff, eine geradkettige oder verzweigte C₁₋₆-Alkylgruppe, eine C₂₋₄-Hydroxyalkylgruppe, eine C₂₋₄-Aminoalkylgruppe, eine Phenylgruppe, die gegebenenfalls mit einer Nitrogruppe, einer Trifluormethylgruppe, einer Aminogruppe oder einer C₁₋₄-Alkylaminogruppe substituiert ist, eine Benzylgruppe, die gegebenenfalls mit einem Halogenatom, einer C₁₋₄-Alkylgruppe, einer C₁₋₄-Alkoxygruppe, einer Methylendioxygruppe oder einer Aminogruppe substituiert ist, eine Pyridylgruppe, eine Thienylgruppe, eine Furylgruppe oder eine Gruppe 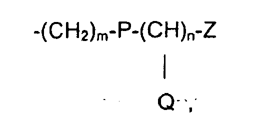 in der m und n unabhängig voneinander ganze Zahlen im Bereich von 1 bis 3 darstellen, P steht für Sauerstoff oder eine Gruppe -NH-, Q steht für Wasserstoff oder eine Methylgruppe und Z steht für eine Methylgruppe, eine Gruppe -OR oder -NRR', wobei R und R' unabhängig voneinander Wasserstoff, eine Methyl- oder eine Ethylgruppe sein können, und für den Fall, dass R¹⁴ Wasserstoff ist, R¹⁵ auch C₁₋₄₋Alkylamino bedeuten kann,
und R¹⁸ steht für eine geradkettige oder verzweigte C₁₋₆-Alkylgruppe, eine C₁₋₄₋Hydroxyalkylgruppe, eine C₁₋₄-Aminoalkylgruppe, eine N-C₁₋₄-Alkyl-C₁₋₄-aminoalkylgruppe, eine N,N-C₁₋₄-Dialkyl-C₁₋₄-aminoalkylgruppe, eine N-C₁₋₄-Hydroxyalkyl-C₁₋₄-aminoalkylgruppe, eine C₁₋₄-Alkoxymethylgruppe, eine Phenylgruppe, die gegebenenfalls mit einem Halogenatom, einer C₁₋₄-Alkylgruppe, einer C₁₋₄-Alkoxygruppe, einer Nitrogruppe, einer Trifluormethylgruppe, einer Aminogruppe oder einer C₁₋₄-Alkylaminogruppe substituiert ist, eine Benzylgruppe, die gegebenenfalls mit einem Halogenatom, einer C₁₋₄-Alkylgruppe, einer C₁₋₄-Alkoxygruppe, einer Aminogruppe, einer Trifluormethylgruppe, einer C₁₋₄-Alkylaminogruppe oder einer Nitrogruppe substituiert ist, einen Heterocyclus, der unter Thiophen, Furan und Pyridin ausgewählt ist, oder eine Gruppe -(CH₂)ₚ-O-(CH₂)_{q}-OR", worin R" für Wasserstoff oder eine Methylgruppe steht und p und q ganze Zahlen sind, die unabhängig voneinander im Bereich von 1 bis 3 liegen, oder für Wasserstoff mit der Maßgabe, dass dann R¹³ von einer unsubstituierten oder substituierten Benzylgruppe verschieden ist,
und mindestens einem halogenierten m-Aminophenol der allgemeinen Formel (I) oder einem seiner physiologisch verträglichen Salze als Kupplerkomponente, 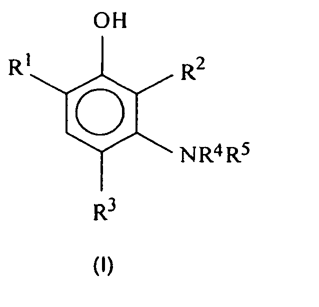 wobei R¹ steht für Wasserstoff oder eine Methylgruppe,
R² steht für Wasserstoff oder ein Halogenatom,
R³ steht für ein Chloratom,
und R⁴ und R⁵ beide für Wasserstoff stehen,
mit der Maßgabe, dass es, wenn das 4,5-Diaminopyrazolderivat 4,5-Diamino-1-hydroxyethylpyrazol ist und die Verbindung der Formel (I) 4-Chlor-2-methyl-5-aminophenol ist, frei ist von N,N'-Bis(4-aminophenyl)-1,4-diazacycloheptan.

Beispiele für die als Substituenten in den erfindungsgemäßen Verbindungen genannten C₁₋₄-Alkylgruppen sind die Gruppen Methyl, Ethyl, Propyl, Isopropyl und Butyl. Ethyl und Methyl sind bevorzugte Alkylgruppen, die Methylgruppe ist besonders bevorzugt. Eine erfindungsgemäß bevorzugte C₁₋₄-Alkoxygruppe ist beispielsweise eine Methoxy- oder eine Ethoxygruppe. Beispiele für eine C₁₋₄-Monohydroxyalkylgruppe sind eine Hydroxymethyl- oder eine Hydroxyethyl-Gruppe. Eine 2-Hydroxyethyl-Gruppe ist besonders bevorzugt. Beispiele für ein Halogenatom sind erfindungsgemäß ein Fluor-, ein Chlor-, ein Brom oder ein Iodatom, ein Chloratom ist besonders bevorzugt. Ein Beispiel für eine mit 1 - 4 Halogenatomen, insbesondere Fluoratomen, substituierten C₁₋₄-Alkylgruppe ist eine Methylgruppe, die ein bis drei Halogenatome, insbesondere Fluoratome, als Substituenten trägt. Erfindungsgemäß besonders bevorzugt sind halogenierte C₁₋₄-Alkylgruppen mit endständiger Trifluormethylgruppe.

Besonders bevorzugt sind erfindungsgemäß solche Verbindungen, bei denen R² für Wasserstoff oder Chlor steht.

Im Sinne der Gesamterfindung hervorragend geeignete Kupplerkomponenten sind 2,4-Dichlor-3-aminophenol und 4-Chlor-2-methyl-5-aminophenol.

Die erfindungsgemäßen Entwicklerkomponenten sind vorzugsweise ausgewählt aus 4,5-Diamino-pyrazol, 4,5-Diamino-1-(2'-hydroxyethyl)-pyrazol, 1-Benzyl-4,5-diamino-3-methyl-pyrazol, 4,5-Diamino-1-(2'-hydroxyethyl)-3-(4'-methoxyphenyl)-pyrazol, 4,5-Diamino-1-(2'-hydroxyethyl)-3-(4'-methylphenyl)-pyrazol, 4,5-Diamino-1-(2'-hydroxyethyl)-3-(3'-methylphenyl)-pyrazol, 4,5-Diamino-3-methyl-1-isopropyl-pyrazol, 4,5-Diamino-3-(4'-methoxyphenyl)-1-isopropyl-pyrazol, 4,5-Diamino-1-ethyl-3-methyl-pyrazol, 4,5-Diamino-1-ethyl-3-(4'-methoxyphenyl)-pyrazol, 4,5-Diamino-3-hydroxymethy-1-methyl-pyrazol, 4,5-Diamino-1-ethyl-3-hydroxymethyl-pyrazol, 4,5-Diamino-3-hydroxymethyl-1-isopropyl-pyrazol, 4,5-Diamino-3-hydroxymethyl-1-tert-butyl-pyrazol, 4,5-Diamino-3-hydroxymethyl-1-phenyl-pyrazol, 4,5-Diamino-3-hydroxymethyl-1-(2'-methoxyphenyl)-pyrazol, 4,5-Diamino-3-hydroxymethyl-1-(3'-methoxyphenyl)-pyrazol, 4,5-Diamino-3-hydroxymethyl-1-(4'-methoxyphenyl)-pyrazol, 1-Benzyl-4,5-diamino-3-hydroxymethyl-pyrazol, 4,5-Diamino-3-methyl-1-(2'-methoxyphenyl)-pyrazol, 4,5-Diamino-3-methyl-1-(3'-methoxyphenyl)-pyrazol, 4,5-Diamino-3-methyl-1-(4'-methoxyphenyl)-pyrazol, 3-Aminomethyl-4,5-diamino-1-methyl-pyrazol, 3-Aminomethyl-4,5-diamino-1-ethyl-pyrazol, 3-Aminomethyl-4,5-diamino-1-isopropyl-pyrazol, 3-Aminomethyl-4,5-diamino-1-tert-butyl-pyrazol, 4,5-Diamino-3-dimethylaminomethyl-1-methyl-pyrazol, 4,5-Diamino-3-dimethylaminomethyl-1-ethyl-pyrazol, 4,5-Diamino-3-dimethylaminomethyl-1-isopropyl-pyrazol, 4,5-Diamino-3-dimethylaminomethyl-1-tert-butyl-pyrazol, 4,5-Diamino-3-ethylaminomethyl-1-methyl-pyrazol, 4,5-Diamino-3-ethylaminomethyl-1-ethyl-pyrazol, 4,5-Diamino-3-ethylaminomethyl-1-isopropyl-pyrazol, 4,5-Diamino-3-ethylaminomethyl-1-tert-butyl-pyrazol, 4,5-Diamino-3-methylaminomethyl-1-methyl-pyrazol, 4,5-Diamino-3-methylaminomethyl-1-isopropyl-pyrazol, 4,5-Diamino-1-ethyl-3-methylaminomethyl-pyrazol, 1-tert-Butyl-4,5-Diamino-3-methylaminomethyl-pyrazol, 4,5-Diamino-3-[(2'-hydroxyethyl)aminomethyl]-1-methyl-pyrazol, 4,5-Diamino-3-[(2'-hydroxyethyl)aminomethyl]-1-isopropyl-pyrazol, 4,5-Diamino-1-ethyl-3-[(2'-hydroxyethyl)aminomethyl]-pyrazol, 1-tert-Butyl-4,5-diamino-3-[(2'-hydroxyethyl)aminomethyl]-pyrazol, 4-Amino-5-(2'-hydroxyethyl)-amino-1,3-dimethyl-pyrazol, 4-Amino-5-(2'-hydroxyethyl)amino-1-isopropyl-3-methyl-pyrazol, 4-Amino-5-(2'-hydroxyethyl)amino-1-ethyl-3-methyl-pyrazol, 4-Amino-5-(2'-hydroxyethyl)amino-1-tert-butyl-3-methyl-pyrazol, 4-Amino-5-(2'-hydroxyethyl)amino-1-phenyl-3-methyl-pyrazol, 4-Amino-5-(2'-hydroxyethyl)amino-1-(2-methoxyphenyl)-3-methyl-pyrazol, 4-Amino-5-(2'-hydroxyethyl)amino-1-(3-methoxyphenyl)-3-methyl-pyrazol, 4-Amino-5-(2'-hydroxyethyl)amino-1-(4-methoxyphenyl)-3-methyl-pyrazol, 4-Amino-5-(2'-hydroxyethyl)amino-1-benzyl-3-methyl-pyrazol, 4-Amino-1-ethyl-3-methyl-5-methylamino-pyrazol, 4-Amine-1-tert-butyl-3-methyl-5-methylamino-pyrazol, 4,5-Diamino-1,3-dimethyl-pyrazol, 4,5-Diamino-3-tert-butyl-1-methyl-pyrazol, 4,5-Diamino-1-tert-butyl-3-methyl-pyrazol, 4,5-Diamino-1-methyl-3-phenyl-pyrazol, 4,5-Diamino-1-(2'-hydroxyethyl)-3-methyl-pyrazol, 4,5-Diamino-1-(2'-hydroxyethyl)-3-phenyl-pyrazol, 4,5-Diamino-1-methyl-3-(2'-chlorphenyl)-pyrazol, 4,5-Diamino-1-methyl-3-(4'-chlorphenyl)-pyrazol, 4,5-Diamino-1-methyl-3-(3'-trifluormethylphenyl)-pyrazol, 4,5-Diamino-1,3-diphenyl-pyrazol, 4,5-Diamino-3-methyl-1-phenyl-pyrazol, 4-Amino-1,3-dimethyl-5-phenylamino-pyrazol, 4-Amino-1-ethyl-3-methyl-5-phenylamino-pyrazol, 4-Amino-1,3-dimethyl-5-methylamino-pyrazol, 4-Amino-3-methyl-1-isopropyl-5-methylamino-pyrazol, 4-Amino-3-isobutoxymethyl-1-methyl-5-methylamino-pyrazol, 4-Amino-3-methoxyethoxymethyl-1-methyl-5-methylamino-pyrazol, 4-Amino-3-hydroxymethyl-1-methyl-5-methylamino-pyrazol, 4-Amino-1,3-diphenyl-5-phenylamino-pyrazol, 4-Amino-3-methyl-5-methylamino-1-phenyl-pyrazol, 4-Amino-1,3-dimethyl-5-hydrazino-pyrazol, 5-Amino-3-methyl-4-methylamino-1-phenyl-pyrazol, 5-Amino-1-methyl-4-(N,N-methylphenyl)amino-3-(4'-chlorphenyl)-pyrazol, 5-Amino-3-ethyl-1-methyl-4-(N,N-methylphenyl)amino-pyrazol, 5-Amino-1-methyl-4-(N,N-methylphenyl)amino-3-phenyl-pyrazol, 5-Amino-3-ethyl-4-(N,N-methylphenyl)amino-pyrazol, 5-Amino-4-(N,N-methylphenyl)amino-3-phenyl-pyrazol, 5-Amino-4-(N,N-methylphenyl)amino-3-(4'-methylphenyl)pyrazol, 5-Amino-3-(4'-chlorphenyl)-4-(N,N-methylphenyl)amino-pyrazol, 5-Amino-3-(4'-methoxyphenyl)-4-(N,N-methylphenyl) amino-pyrazol, 4-Amino-5-methylamino-3-phenylpyrazol, 4-Amino-5-ethylamino-3-phenyl-pyrazol, 4-Amino-5-ethylamino-3-(4'-methylphenyl)-pyrazol, 4-Amino-3-phenyl-5-propylamino-pyrazol, 4-Amino-5-butylamino-3-phenyl-pyrazol, 4-Amino-3-phenyl-5-phenylamino-pyrazol, 4-Amino-5-benzylamino-3-phenyl-pyrazol, 4-Amino-5-(4'-chlorphenyl)amino-3-phenyl-pyrazol, 4-Amino-3-(4'-chlorphenyl)-5-phenylamino-pyrazol, 4-Amino-3-(4'-methoxyphenyl)-5-phenylamino-pyrazol, 1-(4'-Chlorbenzyl)-4,5-diamino-3-methyl-pyrazol, 4,5-Diamino-3-hydroxymethyl-1-isopropyl-pyrazol, 4-Amino-1-ethyl-3-methyl-5-methylamino-pyrazol, 4-Amino-5-(2'-aminoethyl)amino-1,3-dimethyl-pyrazol, 4,5-Diamino-1-(4'-methoxybenzyl)-pyrazol, 4,5-Diamino-1-(4'-methylbenzyl)-pyrazol, 4,5-Diamino-1-(4'-chlorbenzyl)-pyrazol, 4,5-Diamino-1-(3'-methoxybenzyl)-pyrazol, 4-Amino-1-methyl-5-methylamino-pyrazol, 4-Amino-5-(2'-hydroxyethyl)amino-1-methylpyrazol, 4,5-Diamino-1-ethyl-pyrazol, 4,5-Diamino-1-isopropyl-pyrazol, 4-Amino-1-(2'-hydroxyethyl)-5-(2'-hydroxyethyl)amino-pyrazol, 4-Amino-1-(2'-hydroxyethyl)-5-methylamino-pyrazol, 4-Amino-5-methylamino-pyrazol, 4,5-Diamino-1-methyl-pyrazol, 4,5-Diamino-1-benzylpyrazol und 4-Amino-1-methyl-5-N,N-dimethylaminopyrazol sowie beliebigen Mischungen der genannten Komponenten.

Erfindungsgemäß bevorzugte Entwicklerkomponenten sind ausgewählt aus 4,5-Diamino-pyrazol, 4,5-Diamino-1-(2'-hydroxyethyl)-pyrazol, 4,5-Diamino-1,3-dimethylpyrazol, 4,5-Diamino-3-methyl-l-phenyl-pyrazol, 4,5-Diamino-1-methyl-3-phenyl-pyrazol, 4-Amino-1,3-dimethyl-5-hydrazino-pyrazol, 1-Benzyl-4,5-diamino-3-methyl-pyrazol, 4,5-Diamino-3-tert-butyl-1-methyl-pyrazol, 4,5-Diamino-1-tert-butyl-3-methyl-pyrazol, 4,5-Diamino-1-(2'-hydroxyethyl)-3-methyl-pyrazol, 4,5-Diamino-1-ethyl-3-methyl-pyrazol, 4,5-Diamino-1-ethyl-3-(4'-methoxyphenyl)-pyrazol, 4,5-Diamino-1-ethyl-3-hydroxymethyl-pyrazol, 4,5-Diamino-3-hydroxmethyl-1-methyl-pyrazol, 4,5-Diamino-3-hydroxymethyl-1-isopropyl-pyrazol, 4,5-Diamino-3-methyl-1-isopropyl-pyrazol und 4-Amino-5-(2'-aminoethyl)amino-1,3-dimethyl-pyrazol sowie beliebigen Mischungen der genannten Komponenten.

Besonders bevorzugt sind erfindungsgemäß die Entwicklerkomponenten 4,5-Diamino-1-(2'-hydroxyethyl)-pyrazol und 4,5-Diaminopyrazol.

Eine im Sinne der Gesamterfindung hervorragend geeignete Entwicklerkomponente ist 4,5-Diamino-1-(2'-hydroxyethyl)-pyrazol.

Da es sich bei allen erfindungsgemäßen Substanzen um Amino-Verbindungen handelt, lassen sich aus diesen in üblicher Weise die bekannten Säureadditionssalze herstellen. Alle Aussagen dieser Schrift und demgemäß der beanspruchte Schutzbereich beziehen sich daher sowohl auf die in freier Form vorliegenden Amino-Verbindungen gemäß den Formeln (I) und (II) als auch auf deren wasserlösliche, physiologisch verträgliche Salze. Beispiele für solche Salze sind die Hydrochloride, die Hydrobromide, die Sulfate, die Phosphate, die Acetate, die Propionate, die Citrate und die Lactate, wobei die Hydrochloride bevorzugt sind.

Die erfindungsgemäßen Kombinationen von Entwickler- und speziellen Kupplerkomponenten ergeben in einer bevorzugten Ausführungsform ein Oxidationsfärbemittel, das beide Komponenten in Mengen von jeweils 0,005 bis 20 Gew.-%, besonders bevorzugt jeweils 0,1 bis 5 Gew.-%, bezogen auf das gesamte Färbemittel, enthält.

Ein zweiter Gegenstand der vorliegenden Erfindung ist ein Verfahren zum Färben von Keratinfasern, bei dem ein erfindungsgemäßes Oxidationsfärbemittel in einer geeigneten kosmetischen Zubereitung auf die zu färbenden Fasern aufgebracht und die Farbe mit Hilfe eines Oxidationsmittels entwickelt wird, indem das Oxidationsmittel entweder unmittelbar vor der Anwendung zum Färbemittel zugegeben und dann auf die Fasern aufgetragen oder aber gleichzeitig mit dem Färbemittel oder aber direkt nach dem Färbemittel auf die zu färbenden Fasern aufgetragen wird, danach die gesamte Zubereitung etwa 10 bis 45 Minuten, vorzugsweise 30 Minuten lang, auf den Fasern bleibt und anschließend wieder ausgespült oder mit einem Shampoo ausgewaschen wird.

Unter Keratinfasern sind erfindungsgemäß Pelze, Wolle, Federn und insbesondere menschliche Haare zu verstehen. Obwohl die erfindungsgemäßen Kombinationen von Kuppler- und Entwicklerkomponenten in erster Linie als Oxidationsfärbemittel zum Färben von Keratinfasern geeignet sind, steht prinzipiell einer Verwendung auch auf anderen Gebieten, insbesondere in der Farbphotographie, nichts entgegen.

Die erfindungsgemäßen Oxidationsfärbemittel enthalten die erfindungsgemäßen Entwickler- und Kupplerkomponenten und können gewünschtenfalls noch weitere Entwickler- oder Kupplerkomponenten enthalten.

Als Entwicklerkomponenten werden üblicherweise primäre aromatische Amine mit einer weiteren, in para- oder ortho-Position befindlichen, freien oder substituierten Hydroxy- oder Aminogruppe, Diaminopyridinderivate, heterocyclische Hydrazone, sowie 2,4,5,6-Tetraaminopyrimidin und dessen Derivate eingesetzt.

Erfindungsgemäß bevorzugte Entwicklerkomponenten sind p-Phenylendiamin, p-Toluylendiamin, p-Aminophenol, o-Aminophenol, 1-(2'-Hydroxyethyl)-2,5-diaminobenzol, N,N-Bis-(2-hydroxy-ethyl)-p-phenylendiamin, 2-(2,5-Diaminophenoxy)-ethanol, 4-Amino-3-methylphenol, 2,4,5,6-Tetraaminopyrimidin, 2-Hydroxy-4,5,6-triaminopyrimidin, 4-Hydroxy-2,5,6-triaminopyrimidin, 2,4-Dihydroxy-5,6-diaminopyrimidin, 2-Dimethylamino-4,5,6-triaminopyrimidin, 2-Hydroxymethylamino-4-amino-phenol, Bis-(4-aminophenyl)amin, 4-Amino-3-fluorphenol, 2-Aminomethyl-4-aminophenol, 2-Hydroxymethyl-4-aminophenol, 4-Amino-2-((diethylamino)-methyl)-phenol, Bis-(2-hydroxy-5-aminophenyl)-methan, 1,4-Bis-(4-aminophenyl)-diazacycloheptan, 1,3-Bis(N(2'-hydroxyethyl)-N(4-aminophenylamino))-2-propanol, 4-Amino-2-(2'-hydroxyethoxy)-phenol sowie 1,10-Bis-(2,5-diaminophenyl)-1,4,7,10-tetraoxadecan.

Weitere besonders bevorzugte Entwicklerkomponenten sind p-Phenylendiamin, p-Toluylendiamin, p-Aminophenol, 1-(2'-Hydroxyethyl)-2,5-diaminobenzol, N,N-Bis-(2'-hydroxy-ethyl)-p-phenylendiamin, 4-Amino-3-methylphenol, 4-Amino-2-((diethylamino)-methyl)-phenol, 2-Aminomethyl-4-aminophenol, 2,4,5,6-Tetraaminopyrimidin, 2-Hydroxy-4,5,6-triaminopyrimidin und 4-Hydroxy-2,5,6-triaminopyrimidin.

Erfindungsgemäß bevorzugte Kupplerkomponenten sind
- m-Aminophenol und dessen Derivate wie beispielsweise 5-Amino-2-methylphenol, 2-Hydroxy-4-aminophenoxyethanol, 2,6-Dimethyl-3-aminophenol, 5-Amino-4-methoxy-2-methylphenol, 5-(2'-Hydroxyethyl)-amino-2-methylphenol, 3-(Diethylamino)-phenol, N-Cyclopentyl-3-aminophenol, 1,3-Dihydroxy-5-(methylamino)-benzol und 3-(Ethylamino)-4-methylphenol,
- o-Aminophenol und dessen Derivate,
- m-Diaminobenzol und dessen Derivate wie beispielsweise 2,4-Diaminophenoxyethanol, 1,3-Bis-(2,4-diaminophenoxy)-propan, 1-Methoxy-2-amino-4-(2'-hydroxyethylamino)benzol, 1,3-Bis-(2,4-diaminophenyl)-propan, 2,6-Bis-(2'-hydroxyethylamino)-1-methylbenzol und 1-Amino-3-bis-(2'-hydroxyethyl)-aminobenzol,
- o-Diaminobenzol und dessen Derivate wie beispielsweise 3,4-Diaminobenzoesäure und 2,3-Diamino-1-methylbenzol,
- Di- beziehungsweise Trihydroxybenzolderivate wie beispielsweise Resorcin, Resorcinmonomethylether, 2-Methylresorcin, 5-Methylresorcin, 2,5-Dimethylresorcin, 2-Chlorresorcin, 4-Chlorresorcin, Pyrogallol und 1,2,4-Trihydroxybenzol,
- Pyridinderivate wie beispielsweise 2,6-Dihydroxypyridin, 2-Amino-3-hydroxypyridin, 2-Amino-5-chlor-3-hydroxypyridin, 3-Amino-2-methylamino-6-methoxypyridin, 2,6-Dihydroxy-3,4-dimethylpyridin, 2,6-Dihydroxy-4-methylpyridin, 2,6-Diaminopyridin, 2,3-Diainino-6-methoxypyridin und 3,5-Diamino-2,6-dimethoxypyridin,
   Naphthalinderivate wie beispielsweise 1-Naphthol, 2-Methyl-1-naphthol, 2-Hydroxymethyl-1-naphthol, 2-Hydroxyethyl-1-naphthol, 1,5-Dihydroxynaphthalin, 1,6-Dihydroxynaphthalin, 1,7-Dihydroxynaphthalin, 1,8-Dihydroxynaphthalin, 2,7-Dihydroxynaphthalin und 2,3-Dihydroxynaphthalin,
- Morpholinderivate wie beispielsweise 6-Hydroxybenzomorpholin und 6-Aminobenzomorpholin,
- Chinoxalinderivate wie beispielsweise 6-Methyl-1,2,3,4-tetrahydrochinoxalin,
- Pyrazolderivate wie beispielsweise 1-Phenyl-3-methylpyrazol-5-on,
- Indolderivate wie beispielsweise 4-Hydroxyindol, 6-Hydroxyindol und 7-Hydroxyindol,
   Methylendioxybenzolderivate wie beispielsweise 1-Hydroxy-3,4-methylendioxybenzol, 1-Amino-3,4-methylendioxybenzol und 1-(2'-Hydroxyethyl)-amino-3,4-methylendioxybenzol.

Besonders bevorzugte Kupplerkomponenten sind 1-Naphthol, 1,5-, 2,7- und 1,7-Dihydroxynaphthalin, 3-Aminophenol, 5-Amino-2-methylphenol, 2-Amino-3-hydroxypyridin, Resorcin, 4-Chlorresorcin, 2-Chlor-6-methyl-3-aminophenol, 2-Methylresorcin, 5-Methylresorcin, 2,5-Dimethylresorcin und 2,6-Dihydroxy-3,4-dimethylpyridin.

In einer bevorzugten Ausführungsform sind die Entwicklerkomponenten in einer Menge von 0,005 bis 20 Gew.-%, vorzugsweise 0,1 bis 5 Gew.-%, und die Kupplerkomponenten in einer Menge von 0,005 bis 20 Gew.-%, vorzugsweise 0,1 bis 5 Gew.-%, jeweils bezogen auf das gesamte Oxidationsfärbemittel, enthalten.

Zur Erzielung spezieller Nuancen werden Oxidationsfärbemittel häufig auch in Kombination mit direktziehenden Farbstoffen eingesetzt. Direktziehende Farbstoffe sind üblicherweise Nitrophenylendiamine, Nitroaminophenole, Azofarbstoffe, Anthrachinone oder Indophenole. Bevorzugte direktziehende Farbstoffe sind die unter den internationalen Bezeichnungen bzw. Handelsnamen HC Yellow 2, HC Yellow 4, HC Yellow 5, HC Yellow 6, Basic Yellow 57, HC Orange 1, Disperse Orange 3, HC Red 1, HC Red 3, HC Red 13, HC Red BN, Basic Red 76, HC Blue 2, HC Blue 12, Disperse Blue 3, Basic Blue 7, Basic Blue 99, HC Violet 1, Disperse Violet 1, Disperse Violet 4, Basic Violet 14, Acid Violet 43, Disperse Black 9, Acid Black 52, Basic Brown 16 und Basic Brown 17 bekannten Verbindungen sowie 1,4-Bis-(2'-hydroxyethyl)-amino-2-nitrobenzol, 3-Nitro-4-(2'-hydroxyethyl)-aminophenol, 4-Amino-2-nitrodiphenylamin-2'-carbonsäure, 6-Nitro-1,2,3,4-tetrahydrochinoxalin, 2-Hydroxy-1,4-naphthochinon, Hydroxyethyl-2-nitro-toluidin, Pikraminsäure, 2-Amino-6-chloro-4-nitrophenol, 4-Ethylamino-3-nitrobenzoesäure und 2-Chloro-6-ethylamino-1-hydroxy-4-nitrobenzol. Die erfindungsgemäßen Mittel gemäß dieser Ausführungsform enthalten die direktziehenden Farbstoffe bevorzugt in einer Menge von 0,01 bis 20 Gew.-%, bezogen auf das gesamte Färbemittel.

Weiterhin können die erfindungsgemäßen Zubereitungen auch in der Natur vorkommende Farbstoffe wie beispielsweise Henna rot, Henna neutral, Henna schwarz, Kamillenblüte, Sandelholz, schwarzen Tee, Faulbaumrinde, Salbei, Blauholz, Krappwurzel, Catechu, Sedre und Alkannawurzel enthalten.

Weitere in den erfindungsgemäßen Färbemitteln enthaltene Farbstoffkomponenten können auch Indole und Indoline sowie deren physiologisch verträgliche Salze sein. Bevorzugte Beispiele sind 5,6-Dihydroxyindol, N-Methyl-5,6-dihydroxyindol, N-Ethyl-5,6-dihydroxyindol, N-Propyl-5,6-dihydroxyindol, N-Butyl-5,6-dihydroxyindol, 5,6-Dihydroxyindol-2-carbonsäure, 6-Hydroxyindol, 6-Aminoindol und 4-Aminoindol. Weiterhin bevorzugt sind 5,6-Dihydroxyindolin, N-Methyl-5,6-dihydroxyindolin, N-Ethyl-5,6-dihydroxyindolin, N-Propyl-5,6-dihydroxyindolin, N-Butyl-5,6-dihydroxyindolin, 5,6-Dihydroxyindolin-2-carbonsäure, 6-Hydroxyindolin, 6-Aminoindolin und 4-Aminoindolin.

Es ist nicht erforderlich, dass die Oxidationsfarbstoffvorprodukte oder die direktziehenden Farbstoffe jeweils einheitliche Verbindungen darstellen. Vielmehr können in den erfindungsgemäßen Haarfärbemitteln, bedingt durch die Herstellungsverfahren für die einzelnen Farbstoffe, in untergeordneten Mengen noch weitere Komponenten enthalten sein, soweit diese nicht das Färbeergebnis nachteilig beeinflussen oder aus anderen Gründen, z.B. toxikologischen, ausgeschlossen werden müssen.

Bezüglich der in den erfindungsgemäßen Haarfärbe- und -tönungsmitteln einsetzbaren Farbstoffe wird weiterhin ausdrücklich Bezug genommen auf die Monographie C. Zviak, The Science of Hair Care, Kapitel 7 (Seiten 248-250; direktziehende Farbstoffe) sowie Kapitel 8, (Seiten 264-267; Oxidationsfarbstoffvorprodukte), erschienen als Band 7 der Reihe "Dermatology" (Hrg.: Ch., Culnan und H. Maibach), Verlag Marcel Dekker Inc., New York, Basel, 1986, sowie das "Europäische Inventar der Kosmetik-Rohstoffe", herausgegeben von der Europäischen Gemeinschaft, erhältlich in Diskettenform vom Bundesverband Deutscher Industrie- und Handelsunternehmen für Arzneimittel, Reformwaren und Körperpflegemittel e.V., Mannheim.

Haarfärbemittel, insbesondere wenn die Ausfärbung oxidativ, sei es mit Luftsauerstoff oder anderen Oxidationsmitteln wie Wasserstoffperoxid, erfolgt, werden üblicherweise schwach sauer bis alkalisch, d. h. auf pH-Werte im Bereich von etwa 5 bis 11, eingestellt. Zu diesem Zweck enthalten die Färbemittel Alkalisierungsmittel, üblicherweise Alkali- oder Erdalkalihydroxide, Ammoniak oder organische Amine. Bevorzugte Alkalisierungsmittel sind Monoethanolamin, Monoisopropanolamin, 2-Amino-2-methyl-propanol. 2-Amino-2-methyl-1,3-propandiol, 2-Amino-2-ethyl-1,3-propandiol, 2-Amino-2-methylbutanol und Triethanolamin sowie Alkali- und Erdalkalimetallhydroxidc. Insbesondere Monoethanolamin, Triethanolamin sowie 2-Amino-2-methyl-propanol und 2-Amino-2-methyl-1,3-propandiol sind im Rahmen dieser Gruppe bevorzugt. Auch die Verwendung von ω-Aminosäuren wie ω-Aminocapronsäure als Alkalisierungsmittel ist möglich.

Zur Ausbildung der eigentlichen Haarfarben aus den Oxidationsfarbstoffvorprodukten können übliche Oxidationsmittel, wie insbesondere Wasserstoffperoxid oder dessen Anlagerungsprodukte an Harnstoff, Melamin oder Natriumborat verwendet werden. Die Oxidation mit Luftsauerstoff als einzigem Oxidationsmittel kann allerdings bevorzugt sein. Weiterhin ist es möglich, die Oxidation mit Hilfe von Enzymen durchzuführen, wobei die Enzyme sowohl zur Erzeugung von oxidierenden Per-Verbindungen eingesetzt werden als auch zur Verstärkung der Wirkung einer geringen Menge vorhandener Oxidationsmittel. So können die Enzyme (Enzymklasse 1: Oxidoreduktasen) Elektronen aus geeigneten Entwicklerkomponenten (Reduktionsmittel) auf Luftsauerstoff übertragen. Bevorzugt sind dabei Oxidasen wie Tyrosinase und Laccase, aber auch Glucoseoxidase, Uricase oder Pyruvatoxidase. Weiterhin sei das Vorgehen genannt, die Wirkung geringer Mengen (z. B. 1% und weniger, bezogen auf das gesamte Mittel) Wasserstoffperoxid durch Peroxidasen zu verstärken.

Zweckmäßigerweise wird die Zubereitung des Oxidationsmittels dann unmittelbar vor dem Färben der Haare mit der Zubereitung mit den Farbstoffvorprodukten vermischt. Das dabei entstehende gebrauchsfertige Haarfärbepräparat sollte bevorzugt einen pH-Wert im Bereich von 6 bis 10 aufweisen. Besonders bevorzugt ist die Anwendung der Haarfärbemittel in einem schwach alkalischen Milieu. Die Anwendungstemperaturen können in einem Bereich zwischen 15 und 40 °C, bevorzugt bei der Temperatur der Kopfhaut, liegen. Nach einer Einwirkungszeit von ca. 5 bis 45, insbesondere 15 bis 30, Minuten wird das Haarfärbemittel durch Ausspülen von dem zu färbenden Haar entfernt. Das Nachwaschen mit einem Shampoo entfällt, wenn ein stark tensidhaltiger Träger, z. B. ein Färbeshampoo, verwendet wurde.

Insbesondere bei schwer färbbarem Haar kann die Zubereitung mit den Farbstoffvorprodukten ohne vorherige Vermischung mit der Oxidationskomponente auf das Haar aufgebracht werden. Nach einer Einwirkdauer von 20 bis 30 Minuten wird dann - gegebenenfalls nach einer Zwischenspülung - die Oxidationskomponente aufgebracht. Nach einer weiteren Einwirkdauer von 10 bis 20 Minuten wird dann gespült und gewünschtenfalls nachshampooniert. Bei dieser Ausführungsform wird gemäß einer ersten Variante, bei der das vorherige Aufbringen der Farbstoffvorprodukte eine bessere Penetration in das Haar bewirken soll, das entsprechende Mittel auf einen pH-Wert von etwa 4 bis 7 eingestellt. Gemäß einer zweiten Variante wird zunächst eine Luftoxidation angestrebt, wobei das aufgebrachte Mittel bevorzugt einen pH-Wert von 7 bis 10 aufweist. Bei der anschließenden beschleunigten Nachoxidation kann die Verwendung von sauer eingestellten Peroxidisulfat-Lösungen als Oxidationsmittel bevorzugt sein.

Unabhängig davon, welches der oben genannten Vorgehen im Rahmen des erfindungsgemäßen Verfahrens gewählt wird, kann die Ausbildung der Färbung dadurch unterstützt und gesteigert werden, dass dem Mittel bestimmte Metallionen zugesetzt werden. Solche Metallionen sind beispielsweise Zn²⁺, Cu²⁺ Fe²⁺, Fe³⁺, Mn²⁺, Mn⁴⁺, Li⁺, Mg²⁺. Ca²⁺ und Al³⁺. Besonders geeignet sind dabei Zn²⁺, Cu²⁺ und Mn²⁺. Die Metallionen können prinzipiell in der Form eines beliebigen, physiologisch verträglichen Salzes eingesetzt werden. Bevorzugte Salze sind die Acetate, Sulfate, Halogenide, Lactate und Tartrate. Durch Verwendung dieser Metallsalze kann sowohl die Ausbildung der Färbung beschleunigt als auch die Farbnuance gezielt beeinflußt werden.

Zur Herstellung der erfindungsemäßen Färbemittel werden die Oxidationsfarbstoffvorprodukte in einen geeigneten wasserhaltigen Träger eingearbeitet. Zum Zwecke der Haarfärbung sind solche Träger z.B. Cremes, Emulsionen, Gele oder auch tensidhaltige schäumende Lösungen, z.B. Shampoos, Schaumaerosole oder andere Zubereitungen, die für die Anwendung auf dem Haar geeignet sind.

Die erfindungsgemäßen Färbemittel können weiterhin alle für solche Zubereitungen bekannten Wirk-, Zusatz- und Hilfsstoffe enthalten. In vielen Fällen enthalten die Färbemittel mindestens ein Tensid, wobei prinzipiell sowohl anionische als auch zwitterionische, ampholytische, nichtionische und kationische Tenside geeignet sind. In vielen Fällen hat es sich aber als vorteilhaft erwiesen, die Tenside aus anionischen, zwitterionischen oder nichtionischen Tensiden auszuwählen.

Als anionische Tenside eignen sich in erfindungsgemäßen Mitteln alle für die Verwendung am menschlichen Körper geeigneten anionischen oberflächenaktiven Stoffe. Diese sind gekennzeichnet durch eine wasserlöslich machende, anionische Gruppe wie z. B. eine Carboxylat-, Sulfat-, Sulfonat- oder Phosphat-Gruppe und eine lipophile Alkylgruppe mit etwa 10 bis 22 C-Atomen. Zusätzlich können im Molekül Glykol- oder Polyglykolether-Gruppen, Ester-, Ether- und Amidgruppen sowie Hydroxylgruppen enthalten sein. Beispiele für geeignete anionische Tenside sind, jeweils in Form der Natrium-, Kalium-, Magnesium- und Ammonium- sowie der Mono-, Di- und Trialkanolammoniumsalze mit 2 oder 3 C-Atomen in der Alkanolgruppe,
- lineare Fettsäuren mit 10 bis 22 C-Atomen (Seifen),
- Ethercarbonsäuren der Formel R-O-(CH₂-CH₂O)ₓ-CH₂-COOH, in der R eine lineare Alkylgruppe mit 10 bis 22 C-Atomen und x = 0 oder 1 bis 16 ist,
- Amidethercarboxylate der Formel [R-NH(-CH₂-CH₂-O)ₙ-CH₂-COO]ₘZ, in der R für einen linearen oder verzweigten, gesättigten oder ungesättigten Acylrest mit 2 bis 29 C-Atomen, n für ganze Zahlen von 1 bis 10, m für die Zahlen 1 oder 2 und Z für ein Kation aus der Gruppe der Alkali- oder Erdalkalimetalle steht,
- Acylsarcoside mit 10 bis 18 C-Atomen in der Acylgruppe,
- Acyltauride mit 10 bis 18 C-Atomen in der Acylgruppe,
- Acylisethionate mit 10 bis 18 C-Atomen in der Acylgruppe,
- Sulfobemsteinsäuremono- und dialkylester mit 8 bis 18 C-Atomen in der Alkylgruppe und Sulfobernsteinsäuremono-alkylpolyoxyethylester mit 8 bis 18 C-Atomen in der Alkylgruppe und 1 bis 6 Oxyethylgruppen,
- lineare Alkansulfonate mit 12 bis 18 C-Atomen,
- lineare Alpha-Olefinsulfonate mit 12 bis 18 C-Atomen,
- Alpha-Sulfofettsäuremethylester von Fettsäuren mit 12 bis 18 C-Atomen,
- Alkylsulfate und Alkylpolyglykolethersulfate der Formel R-O(-CH₂-CH₂O)ₓ-SO₃H, in der R eine bevorzugt lineare Alkylgruppe mit 10 bis 18 C-Atomen und x = 0 oder 1 bis 12 ist,
- gemischte oberflächenaktive Hydroxysulfonate gemäß DE-A-37 25 030,
- sulfatierte Hydroxyalkylpolyethylen- und/oder Hydroxyalkylenpropylenglykolether gemäß DE-A-37 23 354,
- Sulfonate ungesättigter Fettsäuren mit 12 bis 24 C-Atomen und 1 bis 6 Doppelbindungen gemäß DE-A-39 26 344,
- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen,
- Kokosmonoglyceridsulfate.

Bevorzugte anionische Tenside sind Alkylsulfate, Alkylpolyglykolethersulfate und Ethercarbonsäuren mit 10 bis 18 C-Atomen in der Alkylgruppe und bis zu 12 Glykolethergruppen im Molekül, sowie Sulfobemsteinsäuremono- und -dialkylester mit 8 bis 18 C-Atomen in der Alkylgruppe und Sulfobernsteinsäuremono-alkylpolyoxyethylester mit 8 bis 18 C-Atomen in der Alkylgruppe und 1 bis 6 Oxyethylgruppen sowie Seifen.

Nichtionogene Tenside enthalten als hydrophile Gruppe z. B. eine Polyolgruppe, eine Polyalkylenglykolethergruppe oder eine Kombination aus Polyol- und Polyglykolethergruppe. Solche Verbindungen sind beispielsweise
- Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C₁₂-C₂₂-Fettsäuremono- und -diester von Anlagerungsprodukten von 1 bis 30 Mol Ethylenoxid an Glycerin,
- C₈-C₂₂-Alkylmono- und -oligoglycoside und deren ethoxylierte Analoga,
- Anlagerungsprodukte von 5 bis 60 Mol Ethylenoxid an Rizinusöl und gehärtetes Rizinusöl,
- Anlagerungsprodukte von Ethylenoxid an Sorbitanfettsäureester sowie
- Anlagerungsprodukte von Ethylenoxid an Fettsäurealkanolamide.

Bevorzugte nichtionische Tenside sind Alkylpolyglykoside der allgemeinen Formel RO-(Z)ₓ. Diese Verbindungen sind durch die folgenden Parameter gekennzeichnet.

Der Alkylrest R enthält 8 bis 22 Kohlenstoffatome und kann sowohl linear als auch verzweigt sein. Bevorzugt sind primäre lineare und in 2-Stellung methylverzweigte aliphatische Reste. Solche Alkylreste sind beispielsweise 1-Octyl, 1-Decyl, 1-Lauryl, 1-Myristyl, 1-Cetyl und 1-Stearyl. Besonders bevorzugt sind 1 -Octyl, 1-Decyl, 1-Lauryl, 1-Myristyl. Bei Verwendung sogenannter "Oxo-Alkohole" als Ausgangsstoffe sind die Alkylketten teilweise verzweigt.

Die erfindungsgemäß verwendbaren Alkylpolyglykoside können beispielsweise nur einen bestimmten Alkylrest R enthalten. Üblicherweise werden diese Verbindungen aber ausgehend von natürlichen Fetten und Ölen oder Mineralölen hergestellt. In diesem Fall liegen als Alkylreste R Mischungen entsprechend den Ausgangsverbindungen bzw. entsprechend der jeweiligen Aufarbeitung dieser Verbindungen vor.

Besonders bevorzugt sind solche Alkylpolyglykoside, bei denen R
- im wesentlichen aus C₈- und C₁₀-Alkylgruppen,
- im wesentlichen aus C₁₂- und C₁₄-Alkylgruppen,
- im wesentlichen aus C₈- bis C₁₆-Alkylgruppen oder
- im wesentlichen aus C₁₂- bis C₁₆-Alkylgruppen besteht.

Als Zuckerbaustein Z können beliebige Mono- oder Oligosaccharide eingesetzt werden. Üblicherweise werden Zucker mit 5 bzw. 6 Kohlenstoffatomen sowie die entsprechenden Oligosaccharide eingesetzt. Solche Zucker sind beispielsweise Glucose, Fructose, Galactose, Arabinose, Ribose, Xylose, Lyxose, Allose, Altrose, Mannose, Gulose, Idose, Talose und Sucrose. Bevorzugte Zuckerbausteine sind Glucose, Fructose, Galactose, Arabinose und Sucrose; Glucose ist besonders bevorzugt.

Die erfindungsgemäß verwendbaren Alkylpolyglykoside enthalten im Schnitt 1,1 bis 5 Zuckereinheiten. Alkylpolyglykoside mit x-Werten von 1,1 bis 1,6, auch Alkyloligoglykoside genannt, sind bevorzugt. Ganz besonders bevorzugt sind Alkyloligoglykoside, bei denen x 1,1 bis 1,4 beträgt.

Die Alkylpoly- oder Alkyloligoglykoside können neben ihrer Tensidwirkung auch dazu dienen, die Fixierung von Duftkomponenten auf dem Haar zu verbessern. Der Fachmann wird also für den Fall, daß eine über die Dauer der Haarbehandlung hinausgehende Wirkung des Parfümöles auf dem Haar gewünscht wird, bevorzugt zu dieser Substanzklasse als weiterem Inhaltsstoff der erfindungsgemäßen Zubereitungen zurückgreifen.

Auch die alkoxylierten Homologen der genannten Alkylpolyglykoside können erfindungsgemäß eingesetzt werden. Diese Homologen können durchschnittlich bis zu 10 Ethylenoxid- und/oder Propylenoxideinheiten pro Alkylglykosideinheit enthalten.

Weiterhin können, insbesondere als Co-Tenside, zwitterionische Tenside verwendet werden. Als zwitterionische Tenside werden solche oberflächenaktive Verbindungen bezeichnet, die im Molekül mindestens eine quartäre Ammoniumgruppe und mindestens eine -COO⁽⁻⁾- oder -SO₃⁽⁻⁾-Gruppe tragen. Besonders geeignete zwitterionische Tenside sind die sogenannten Betaine wie die N-Alkyl-N,N-dimethylammonium-glycinate, beispielsweise das Kokosalkyl-dimethylammonium-glycinat, N-Acyl-aminopropyl-N,N-dimethylammoniumglycinate, das Kokosacylaminopropyl-dimethylammoniumglycinat, und 2-Alkyl-3-carboxylmethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.

Ebenfalls insbesondere als Co-Tenside geeignet sind ampholytische Tenside. Unter ampholytischen Tensiden werden solche oberflächenaktiven Verbindungen verstanden, die außer einer C₈-C₁₈-Alkyl- oder Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C₁₂₋₁₈-Acylsarcosin.

Beispiele für die in den erfindungsgemäßen Haarbehandlungsmitteln verwendbaren kationischen Tenside sind insbesondere quartäre Ammoniumverbindungen, Esterquats und Amidoamine.

Bevorzugte quaternäre Ammoniumverbindungen sind Ammoniumhalogenide, insbesondere Chloride und Bromide, wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid, sowie die unter den INCI-Bezeichnungen Quaternium-27 und Quaternium-83 bekannten Imidazolium-Verbindungen. Die langen Alkylketten der oben genannten Tenside weisen bevorzugt 10 bis 18 Kohlenstoffatome auf.

Bei Esterquats handelt es sich um bekannte Stoffe, die sowohl mindestens eine Esterfunktion als auch mindestens eine quartäre Ammoniumgruppe als Strukturelement enthalten. Bevorzugte Esterquats sind quaternierte Estersalze von Fettsäuren mit Triethanolamin, quaternierte Estersalze von Fettsäuren mit Diethanolalkylaminen und quaternierten Estersalze von Fettsäuren mit 1,2-Dihydroxypropyldialkylaminen. Solche Produkte werden beispielsweise unter den Warenzeichen Stepantex®, Dehyquart® und Armocare® vertrieben. Die Produkte Armocare® VGH-70, ein N,N-Bis(2-Palmitoyloxyethyl)dimethylammoniumchlorid, sowie Dehyquart® F-75 und Dehyquart® AU-35 sind Beispiele für solche Esterquats.
Die Alkylamidoamine werden üblicherweise durch Amidierung natürlicher oder synthetischer Fettsäuren und Fettsäureschnitte mit Dialkylaminoaminen hergestellt. Eine erfindungsgemäß besonders geeignete Verbindung aus dieser Substanzgruppe stellt das unter der Bezeichnung Tegoamid® S 18 im Handel erhältliche Stearamidopropyldimethylamin dar.

Ein Beispiel für ein als kationisches Tensid einsetzbares quaternäres Zuckerderivat stellt das Handelsprodukt Glucquat®100 dar, gemäß INCI-Nomenklatur ein "Lauryl Methyl Gluceth-10 Hydroxypropyl Dimonium Chloride".

Bei den als Tensid eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so daß man Substanzgemische mit unterschiedlichen, vom jeweiligen Rohstoff abhängigen Alkylkettenlängen erhält.

Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder -alkoholate als Katalysatoren verwendet werden. Die Verwendung von Produkten mit eingeengter Homologenverteilung kann bevorzugt sein.

Die erfindungsgemäß verwendeten Oxidatonsfärbemittel enthalten gemäß einer bevorzugten Ausführungsform einen Pflegestoff. Dieser Pflegestoff ist bevorzugt ausgewählt aus der Gruppe, die von kationischen Polymeren, Silikonen und Proteinhydrolysaten sowie deren Derivaten gebildet wird.

Eine erste Gruppe von kationischen Polymeren sind die sogenannten "temporär kationischen" Polymere. Diese Polymere enthalten üblicherweise eine Aminogruppe, die bei bestimmten pH-Werten als quartäre Ammoniumgruppe und somit kationisch vorliegt.

Unter den kationischen Polymeren sind aber die permanent kationischen Polymere bevorzugt. Als "permanent kationisch" werden erfindungsgemäß solche Polymeren bezeichnet, die unabhängig vom pH-Wert des Mittels eine kationische Gruppe aufweisen. Dies sind in der Regel Polymere, die ein quartäres Stickstoffatom, beispielsweise in Form einer Ammoniumgruppe, enthalten. Bevorzugte kationische Polymere sind beispielsweise
- quaternisierte Cellulose-Derivate, wie sie unter den Bezeichnungen Celquat® und Polymer JR® im Handel erhältlich sind. Die Verbindungen Celquat® H 100, Celquat® L 200 und Polymer JR®400 sind bevorzugte quaternierte Cellulose-Derivate,
- Polysiloxane mit quaternären Gruppen, wie beispielsweise die im Handel erhältlichen Produkte Q2-7224 (Hersteller: Dow Corning; ein stabilisiertes Trimethylsilylamodimethicon), Dow Coming® 929 Emulsion (enthaltend ein hydroxyl-amino-modifiziertes Silicon, das auch als Amodimethicone bezeichnet wird), SM-2059 (Hersteller: General Electric), SLM-55067 (Hersteller: Wacker) sowie Abil®-Quat 3270 und 3272 (Hersteller: Th. Goldschmidt; diquaternäre Polydimethylsiloxane, Quaternium-80),
- Kationische Guar-Derivate, wie insbesondere die unter den Handelsnamen Cosmedia®Guar und Jaguar® vertriebenen Produkte,
- Polymere Dimethyldiallylammoniumsalze und deren Copolymere mit Estern und Amiden von Acrylsäure und Methacrylsäure. Die unter den Bezeichnungen Merquat®100 (Poly(dimethyldiallylammoniumchlorid)) und Merquat®550 (Dimethyldiallylammoniumchlorid-Acrylamid-Copolymer) im Handel erhältlichen Produkte sind Beispiele für solche kationischen Polymere,
- Copolymere des Vinylpyrrolidons mit quaternierten Derivaten des Dialkylaminoalkylacrylats und -methacrylats, wie beispielsweise mit Diethylsulfat quaternierte Vinylpyrrolidon-Dimethylaminoethylmethacrylat-Copolymere. Solche Verbindungen sind unter den Bezeichnungen Gafquat®734 und Gafquat®755 im Handel erhältlich,
- Vinylpyrrolidon-Vinylimidazoliummethochlorid-Copolymere, wie sie unter den Bezeichnungen Luviquat® FC 370, FC 550, FC 905 und HM 552 angeboten werden.
- quaternierter Polyvinylalkohol,
- sowie die unter den Bezeichnungen Polyquaternium 2, Polyquaternium 17, Polyquaternium 18 und Polyquaternium 27 bekannten Polymeren mit quartären Stickstoffatomen in der Polymerhauptkette.

Gleichfalls als kationische Polymere eingesetzt werden können die unter den Bezeichnungen Polyquaternium-24 (Handelsprodukt z. B. Quatrisoft® LM 200), Polyquaternium-32, Polyquaternium-35 und Polyquaternium-37 (Handelsprodukte z. B. Salcare® SC 92 und Salcare®SC 95) bekannten Polymere. Ebenfalls erfindungsgemäß verwendbar sind die Copolymere des Vinylpyrrolidons, wie sie als Handelsprodukte Copolymer 845 (Hersteller: ISP), Gaffix®VC 713 (Hersteller: ISP), Gafquat®ASCP 1011, Gafquat®HS 110, Luviquat®8155 und Luviquat® MS 370 erhältlich sind.

Erfindungsgemäß bevorzugte kationische Polymere sind quaternisierte Cellulose-Derivate, polymere Dimethyldiallylammoniumsalze, Polyquaternium-27 und deren Copolymere sowie Polymere vom Typ Polyquaterniuim-2. Kationische Cellulose-Derivate, insbesondere das Handelsprodukt Polymer®JR 400, und Polymere vom Typ Polyquaternium-2, insbesondere das Handelsprodukt Mirapol®A-15, sind ganz besonders bevorzugte kationische Polymere.

Die kationischen Polymeren sind in den erfindungsgemäß verwendeten Mitteln bevorzugt in Mengen von 0,05 bis 10 Gew.-%, bezogen auf das gesamte Mittel, enthalten. Mengen von 0,1 bis 5 Gew.-% sind besonders bevorzugt.

Geeignet als Pflegestoff in Kombination mit oder alternativ zu kationischen Polymeren sind auch Ampho-Polymere. Unter dem Oberbegriff Ampho-Polymere sind amphotere Polymere, d. h. Polymere, die im Molekül sowohl freie Aminogruppen als auch freie - COOH- oder SO₃H-Gruppen enthalten und zur Ausbildung innerer Salze befähigt sind, zwitterionische Polymere, die im Molekül quartäre Ammoniumgruppen und -COOoder -SO₃⁻-Gruppen enthalten, und solche Polymeren zusammengefaßt, die -COOH- oder SO₃H-Gruppen und quartäre Ammoniumgruppen enthalten. Ein Beispiel für ein erfindungsgemäß einsetzbares Amphopolymer ist das unter der Bezeichnung Amphomer® erhältliche Acrylharz, das ein Copolymeres aus tert-Butylaminoethylmethacrylat, N-(1,1,3,3-Tetramethylbutyl)acrylamid sowie zwei oder mehr Monomeren aus der Gruppe Acrylsäure, Methacrylsäure und deren einfachen Estern darstellt. Ebenfalls bevorzugte Amphopolymere setzen sich aus ungesättigten Carbonsäuren (z. B. Acryl- und Methacrylsäure), kationisch derivatisierten ungesättigten Carbonsäuren (z. B. Acrylamidopropyl-trimethyl-ammoniumchlorid) und gegebenenfalls weiteren ionischen oder nichtionogenen Monomeren zusammen, wie beispielsweise der deutschen Offenlegungsschrift 39 29 973 und dem dort zitierten Stand der Technik zu entnehmen ist. Terpolymere von Acrylsäure, Methylacrylat und Methacrylamidopropyltrimoniumchlorid, wie sie unter der Bezeichnung Merquat®2001 N im Handel erhältlich sind, sind erfindungsgemäß besonders bevorzugte Ampho-Polymere.

Erfindungsgemäß verwendbare Pflegestoffe sind weiterhin Silikonöle und Silikon-Gums, insbesondere Dialkyl- und Alkylarylsiloxane, wie beispielsweise Dimethylpolysiloxan und Methylphenylpolysiloxan, sowie deren alkoxylierte und quaternierte Analoga. Beispiele für solche Silikone sind die von Dow Coming unter den Bezeichnungen DC 190, DC 200 und DC 1401 vertriebenen Produkte sowie die Handelsprodukte DC 344 und DC 345 von Dow Coming, Q2-7224 (Hersteller: Dow Corning; ein stabilisiertes Trimethylsilylamodimethicon), Dow Corning® 929 Emulsion (enthaltend ein hydroxyl-amino-modifiziertes Silicon, das auch als Amodimethicone bezeichnet wird), SM-2059 (Hersteller: General Electric), SLM-55067 (Hersteller: Wacker) sowie Abil®-Quat 3270 und 3272 (Hersteller: Th. Goldschmidt; diquaternäre Polydimethylsiloxane, Quaternium-80) und das Handelsprodukt Fancorsil® LIM-1. Ein geeignetes anionisches Silikonöl ist das Produkt Dow Corning®1784.

Proteinhydrolysate sind Produktgemische, die durch sauer, basisch oder enzymatisch katalysierten Abbau von Proteinen (Eiweißen) erhalten werden.

Erfindungsgemäß können Proteinhydrolysate sowohl pflanzlichen als auch tierischen Ursprungs eingesetzt werden.

Tierische Proteinhydrolysate sind beispielsweise Elastin-, Kollagen-, Keratin-, Seiden- und Milcheiweiß-Proteinhydrolysate, die auch in Form von Salzen vorliegen können. Solche Produkte werden beispielsweise unter den Warenzeichen Dehylan® (Henkel), Promois® (Interorgana), Collapuron® (Henkel), Nutrilan® (Grünau), Gelita-Sol® (Deutsche Gelatine Fabriken Stoess & Co), Lexein® (Inolex) und Kerasol® (Croda) vertrieben.

Erfindungsgemäß bevorzugt ist die Verwendung von Proteinhydrolysaten pflanzlichen Ursprungs, z. B. Soja-, Mandel-, Erbsen-, Kartoffel- und Weizenproteinhydrolysate. Solche Produkte sind beispielsweise unter den Warenzeichen Gluadin® (Henkel), DiaMin® (Diamalt), Lexein® (Inolex) und Crotein® (Croda) erhältlich.

Wenngleich der Einsatz der Proteinhydrolysate als solche bevorzugt ist, können an deren Stelle gegebenenfalls auch anderweitig erhaltene Aminosäuregemische eingesetzt werden. Ebenfalls möglich, wenngleich weniger bevorzugt, ist der Einsatz von Derivaten der Proteinhydrolysate, beispielsweise in Form ihrer Fettsäure-Kondensationsprodukte oder kationisch derivatisiert. Solche Produkte werden beispielsweise unter den Bezeichnungen Lamepon® (Henkel), Lexein® (Inolex), Crolastin® (Croda), Crotein® (Croda), Lamequat® und Croquat® vertrieben.

Die Proteinhydrolysate oder deren Derivate sind in den erfindungsgemäß verwendeten Oxidationsfärbemitteln bevorzugt in Mengen von 0,1 bis 5 Gew.-%, bezogen auf das gesamte Mittel, enthalten. Mengen von 0,1 bis 2 Gew.-% sind besonders bevorzugt.

Neben dem Oxidationsmittel und den weiteren, oben genannten bevorzugten Komponenten können die Oxidationsfärbemittel prinzipiell alle weiteren, dem Fachmann für solche kosmetischen Mittel bekannten Komponenten enthalten.

Weitere Wirk-, Hilfs- und Zusatzstoffe sind beispielsweise
- Verdickungsmittel wie Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie z. B. Bentonit oder vollsynthetische Hydrokolloide wie z. B. Polyvinylalkohol,
- Strukturanten wie Maleinsäure und Milchsäure,
- haarkonditionierende Verbindungen wie Phospholipide, beispielsweise Sojalecithin, Ei-Lecitin und Kephaline,
- Parfümöle, Dimethylisosorbid und Cyclodextrine,
- Lösungsmittel und -vermittler wie Ethanol, Isopropanol, Ethylenglykol, Propylenglykol, Glycerin und Diethylenglykol,
- faserstrukturverbessemde Wirkstoffe, insbesondere Mono-, Di- und Oligosaccharide wie beispielsweise Glucose, Galactose, Fructose, Fruchtzucker und Lactose,
- Antischuppenwirkstoffe wie Piroctone Olamine, Zink Omadine und Climbazol,
- Lichtschutzmittel, insbesondere derivatisierte Benzophenone, Zimtsäure-Derivate und Triazine,
- Substanzen zur Einstellung des pH-Wertes, wie beispielsweise übliche Säuren, insbesondere Genußsäuren und Basen,
- Wirkstoffe wie Panthenol, Pantothensäure, Allantoin, Pyrrolidoncarbonsäuren und deren Salze, Bisabolol, Pflanzenextrakte und Vitamine
- Cholesterin,
- Konsistenzgeber wie Zuckerester, Polyolester oder Polyolalkylether,
- Fette und Wachse wie Walrat, Bienenwachs, Montanwachs und Paraffine,
- Fettsäurealkanolamide,
- Komplexbildner wie EDTA, NTA und Phosphonsäuren,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate,
- Trübungsmittel wie Latex,
- Perlglanzmittel wie Ethylenglykolmono- und -distearat sowie PEG-3-distearat,
- Stabilisierungsmittel für Wassserstoffperoxid und andere Oxidationsmittel,
- Treibmittel wie Propan-Butan-Gemische, N₂O, Dimethylether, CO₂ und Luft.

Bezüglich weiterer fakultativer Komponenten sowie die eingesetzten Mengen dieser Komponenten wird ausdrücklich auf die dem Fachmann bekannten einschlägigen Handbücher, z. B. K. H. Schrader, Grundlagen und Rezepturen der Kosmetika, 2. Auflage, Hüthig Buch Verlag, Heidelberg, 1989, verwiesen.

Die nachfolgenden Beispiele sollen den Erfindungsgegenstand näher erläutern, ohne ihn auf die Beispiele zu beschränken.

### Beispiele

### 1. Ausführungsbeispiele der Ausfärbungen

Alle Mengenangaben in den Beispielen sind Gewichtsteile.

### 1.1 Herstellung der Färbecreme

### Teilmischung A

| | |
|---|---|
| Hydrenol® D¹ | 8,50g |
| Lorol® techn.² | 2,00g |
| Eumulgin® B2³ | 0,75g |
| Texapon® NSO⁴ | 20,00g |
| Dehyton® K⁵ | 12,50g |
| Wasser | 30,00g |

| | |
|---|---|
| ¹ C₁₆₋₁₈-Fettalkohol (INCI-Bezeichnung: Cetearyl alcohol) (HENKEL) ² C₁₂₋₁₈-Fettalkohol (INCI-Bezeichnung: Coconut alcohol) (HENKEL) ³ Cetylstearylalkohol mit ca. 20 EO-Einheiten (INCI-Bezeichnung: Ceteareth-20) (HENKEL) ⁴ Laurylethersulfat, Natriumsalz (ca. 27,5% Aktivsubstanz; INCI-Bezeichnung: Sodium Laureth Sulfate) (HENKEL) ⁵ N,N-Dimethyl-N-(C₈₋₁₈-kokosamidopropyl)ammoniumacetobetain (ca. 30% Aktivsubstanz; INCI-Bezeichnung: Aqua (Water), Cocamidopropyl Betaine) (HENKEL) | |

Die Substanzen Hydrenol® D, Lorol® und Eumulgin® B2 wurden bei 80°C aufgeschmolzen, mit dem 80°C heißem Wasser, enthaltend Texapon® NSO und Dehyton® K, vermischt und unter starkem Rühren emulgiert. Danach wurde die Emulsion unter schwachem Rühren abgekühlt.

### Teilmischung B

| | |
|---|---|
| Natriumsulfit | 1,00g |
| Ammoniumsulfat | 1,00g |
| Farbstoffvorprodukte | jeweils 7,5 mmol |
| Ammoniak (25%ige Lösung) | ad pH=10,0 |
| Wasser | 10,00g |

Die Farbstoffvorprodukte wurden in dem 50°C heißem Wasser unter Zugabe von Natriumsulfit, Ammoniumsulfat und Ammoniak gelöst.
Die Farbstoffvorproduktlösung (Teilmischung B) wurde zur Emulsion (Teilmischung A) gegeben, mit Ammoniak auf pH = 10 eingestellt und mit Wasser auf 100 g aufgefüllt. Es wurde bis zum Erreichen der Raumtemperatur weitergerührt.

### 1.2 Färbung der keratinischen Fasern

Die so erhaltene Färbecreme wurde im Verhältnis 2:1 mit einer 3%-igen H₂O₂-Lösung vermischt und auf 5cm lange Strähnen standardisierten, zu 80% ergrauten, aber nicht besonders vorbehandelten Menschenhaares (Kerling) aufgetragen. Nach 30 Minuten Einwirkzeit bei 32°C wurde das Haar gespült, mit einem üblichen Haarwaschmittel ausgewaschen und anschließend getrocknet.

Für die Ausfärbungen wurden folgende Kupplerkomponenten verwendet:
- 2,4-Dichlor-3-aminophenol (K1)
- 4-Chlor-2-methyl-5-aminophenol (K2)

Mit 4,5-Diamino-1-(2'-hydroxyethyl)-pyrazol als Entwickler wurden folgende Ausfärbungen gefunden:

| Kuppler | | Nuance des gefärbten Haares |
|---|---|---|
| K1 | 2,4-Dichlor-3-aminophenol | rubin |
| K2 | 4-Chlor-2-methyl-5-aminophenol | paprikarot |

Die Anfärbungen zeichneten sich durch hohe Waschechtheit und leuchtende Farben aus.

## Patentansprüche

1. Oxidationsfärbemittel zum Färben von keratinhaltigen Fasern, **dadurch gekennzeichnet, dass** es eine Kombination enthält aus mindestens einem 4,5-Diaminopyrazolderivat der allgemeinen Formel (II) oder einem seiner physiologisch verträglichen Salze, 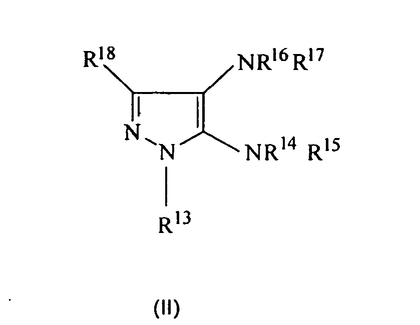 wobei R¹³, R¹⁴, R¹⁵, R¹⁶ und R¹⁷ unabhängig voneinander stehen für Wasserstoff, eine geradkettige oder verzweigte C₁₋₆-Alkylgruppe, eine C₂₋₄-Hydroxyalkylgruppe, eine C₂₋₄-Aminoalkylgruppe, eine Phenylgruppe, die gegebenenfalls mit einer Nitrogruppe, einer Trifluormethylgruppe, einer Aminogruppe oder einer C₁₋₄-Alkylaminogruppe substituiert ist, eine Benzylgruppe, die gegebenenfalls mit einem Halogenatom, einer C₁₋₄-Alkylgruppe, einer C₁₋₄-Alkoxygruppe, einer Methylendioxygruppe oder einer Aminogruppe substituiert ist, eine Pyridylgruppe, eine Thienylgruppe, eine Furylgruppe oder eine Gruppe 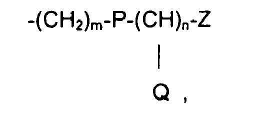 in der m und n unabhängig voneinander ganze Zahlen im Bereich von 1 bis 3 darstellen, P steht für Sauerstoff oder eine Gruppe -NH-, Q steht für Wasserstoff oder eine Methylgruppe und Z steht für eine Methylgruppe, eine Gruppe -OR oder -NRR', wobei R und R' unabhängig voneinander Wasserstoff, eine Methyl- oder eine Ethylgruppe sein können, und für den Fall, dass R¹⁴ Wasserstoff ist, R¹⁵ auch C₁₋₄₋Alkylamino bedeuten kann,
und R¹⁸ steht für eine geradkettige oder verzweigte C₁₋₆-Alkylgruppe, eine C₁₋₄₋Hydroxyalkylgruppe, eine C₁₋₄-Aminoalkylgruppe, eine N-C₁₋₄-Alkyl-C₁₋₄-aminoalkylgruppe, eine N,N-C₁₋₄-Dialkyl-C₁₋₄-aminoalkylgruppe, eine N-C₁₋₄-Hydroxyalkyl-C₁₋₄-aminoalkylgruppe, eine C₁₋₄-Alkoxymethylgruppe, eine Phenylgruppe, die gegebenenfalls mit einem Halogenatom, einer C₁₋₄-Alkylgruppe, einer C₁₋₄-Alkoxygruppe, einer Nitrogruppe, einer Trifluormethylgruppe, einer Aminogruppe oder einer C₁₋₄-Alkylaminogruppe substituiert ist, eine Benzylgruppe, die gegebenenfalls mit einem Halogenatom, einer C₁₋₄-Alkylgruppe, einer C₁₋₄-Alkoxygruppe, einer Aminogruppe, einer Trifluormethylgruppe, einer C₁₋₄-Alkylaminogruppe oder einer Nitrogruppe substituiert ist, einen Heterocyclus, der unter Thiophen, Furan und Pyridin ausgewählt ist, oder eine Gruppe -(CH₂)ₚ-O-(CH₂)_{q}-OR", worin R" für Wasserstoff oder eine Methylgruppe steht und p und q ganze Zahlen sind, die unabhängig voneinander im Bereich von 1 bis 3 liegen, oder für Wasserstoff mit der Maßgabe, dass dann R¹³ von einer unsubstituierten oder substituierten Benzylgruppe verschieden ist,
und mindestens einem halogenierten m-Aminophenol der allgemeinen Formel (I) oder einem seiner physiologisch verträglichen Salze als Kupplerkomponente, 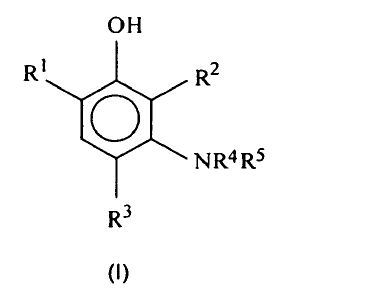 wobei R¹ steht für Wasserstoff oder eine Methylgruppe,
R² steht für Wasserstoff oder ein Halogenatom,
R³ steht für ein Chloratom,
und R⁴ und R⁵ beide für Wasserstoff stehen,
mit der Maßgabe, dass es, wenn das 4,5-Diaminopyrazolderivat 4,5-Diamino-1-hydroxyethylpyrazol ist und die Verbindung der Formel (I) 4-Chlor-2-methyl-5-aminophenol ist, frei ist von N,N'-Bis(4-aminophenyl)-1,4-diazacycloheptan.

2. Oxidationsfärbemittel nach Anspruch 1, **dadurch gekennzeichnet, dass** R² Wasserstoff oder ein Chloratom ist.

3. Oxidationsfärbemittel nach einem der Ansprüche 1 oder 2, **dadurch gekennzeichnet, dass** das halogenierte m-Aminophenol aus 2,4-Dichlor-3-aminophenol und 4-Chlor-2-methyl-5-aminophenol oder einem der physiologisch verträglichen Salze dieser Verbindungen ausgewählt ist.

4. Oxidationsfärbemittel nach einem der Ansprüche 1 bis 3, **dadurch gekennzeichnet, dass** es als Entwicklerkomponente mindestens ein 4,5-Diaminopyrazolderivat, ausgewählt aus 4,5-Diaminopyrazol, 4,5-Diamino-1-(2'-hydroxyethyl)-pyrazol, 4,5-Diamino-1,3-dimethyl-pyrazol, 4,5-Diamino-3-methyl-1-phenyl-pyrazol, 4,5-Diamino-1-methyl-3-phenyl-pyrazol, 1-Benzyl-4,5-diamino-3-methyl-pyrazol, 4,5-Diamino-1-tert-butyl-3-methyl-pyrazol, 4,5-Diamino-1-(2'-hydroxyethyl)-3-methyl-pyrazol, 4,5-Diamino-1-ethyl-3-methyl-pyrazol, 4,5-Diamino-1-ethyl-3-(4'-methoxyphenyl)-pyrazol, 4,5-Diamino-1-ethyl-3-hydroxymethyl-pyrazol, 4,5-Diamino-3-hydroxymethyl-1-methyl-pyrazol, 4,5-Diamino-3-hydroxymethyl-1-isopropyl-pyrazol, 4,5-Diamino-3-methyl-1-isopropyl-pyrazol oder einem der physiologisch verträglichen Salze dieser Verbindungen enthält.

5. Oxidationsfärbemittel nach Anspruch 4, **dadurch gekennzeichnet, dass** es mindestens eine Entwicklerkomponente, ausgewählt aus 4,5-Diaminopyrazol, 4,5-Diamino-1-(2'-hydroxyethyl)-pyrazol oder einem der physiologisch verträglichen Salze dieser Verbindungen enthält.

6. Oxidationsfärbemittel nach einem der Ansprüche 1 bis 5, **dadurch gekennzeichnet, dass** es mindestens eine weitere Entwicklerkomponente enthält.

7. Oxidationsfärbemittel nach Anspruch 6, **dadurch gekennzeichnet, dass** eine weitere Entwicklerkomponente, ausgewählt aus p-Phenylendiamin, p-Toluylendiamin, p-Aminophenol, 1-(2'-Hydroxyethyl)-2,5-diaminobenzol, N,N-Bis-(2'-hydroxy-ethyl)-p-phenylendiamin, 4-Amino-3-methylphenol, 4-Amino-2-((diethylamino)-methyl)-phenol, 2-Aminomethyl-4-aminophenol, 2,4,5,6-Tetraaminopyrimidin, 2-Hydroxy-4,5,6-triaminopyrimidin und 4-Hydroxy-2,5,6-triaminopyrimidin enthalten ist.

8. Oxidationsfärbemittel nach einem der Ansprüche 1 bis 7, **dadurch gekennzeichnet, dass** es mindestens eine weitere Kupplerkomponente enthält.

9. Oxidationsfärbemittel nach Anspruch 8, **dadurch gekennzeichnet, dass** es mindestens eine Kuppierkomponente, ausgewählt aus 1-Naphthol, 1,5-, 2,7- und 1,7-Dihydroxynaphthalin, 3-Aminophenol, 5-Amino-2-methylphenol, 2-Chlor-6-methyl-3-aminophenol, 2-Amino-3-hydroxypyridin, Resorcin, 4-Chlorresorcin, 2-Methylresorcin, 5-Methylresorcin, 2,5-Dimethylresorcin oder 2,6-Dihydroxy-3,4-dimethylpyridin enthält.

10. Oxidationsfärbemittel nach einem der Ansprüche 1 bis 9, **dadurch gekennzeichnet, dass** Entwicklerkomponenten in einer Menge von 0,005 bis 20 Gew.-%, vorzugsweise 0,1 bis 5 Gew.-%, und Kupplerkomponenten in einer Menge von 0,005 bis 20 Gew.-%, vorzugsweise 0,1 bis 5 Gew.-%, jeweils bezogen auf das gesamte Oxidationsfärbemittel, enthalten sind.

11. Oxidationsfärbemittel nach einem der Ansprüche 1 bis 10, **dadurch gekennzeichnet, dass** weiterhin mindestens ein direktziehender Farbstoff enthalten ist.

12. Verfahren zum Färben von Keratinfasern, **dadurch gekennzeichnet, dass** ein Oxidationsfärbemittel nach einem der Ansprüche 1 bis 11 in einer geeigneten kosmetischen Zubereitung auf die zu färbenden Fasern aufgebracht und die Farbe mit Hilfe eines Oxidationsmittels entwickelt wird, indem das Oxidationsmittel entweder unmittelbar vor der Anwendung zum Färbemittel zugegeben und dann auf die Fasern aufgetragen oder aber gleichzeitig mit dem Färbemittel oder aber direkt nach dem Färbemittel auf die zu färbenden Fasern aufgetragen wird, danach die gesamte Zubereitung etwa 10 bis 45 Minuten, vorzugsweise 30 Minuten lang, auf den Fasern bleibt und anschließend wieder ausgespült oder mit einem Shampoo ausgewaschen wird.

## Claims

1. Oxidation colorant for dyeing keratin-containing fibres, **characterized in that** it comprises a combination of at least one 4,5-diaminopyrazole derivative of the general formula (II) or one of its physiologically compatible salts, 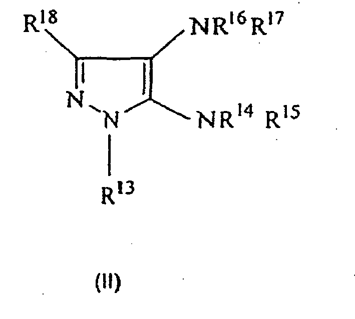 where R¹³, R¹⁴, R¹⁵, R¹⁶ and R¹⁷, independently of one another, are hydrogen, a straight-chain or branched C₁₋₆-alkyl group, a C₂₋₄-hydroxyalkyl group, a C₂₋₄-aminoalkyl group, a phenyl group, which is optionally substituted by a nitro group, a trifluoromethyl group, an amino group or a C₁₋₄-alkylamino group, a benzyl group, which is optionally substituted by a halogen atom, a C₁₋₄-alkyl group, a C₁₋₄-alkoxy group, a methylenedioxy group or an amino group, a pyridyl group, a thienyl group, a furyl group or a group 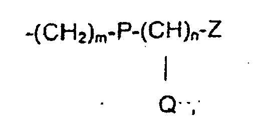 in which m and n, independently of one another, are integers in the range from 1 to 3, P is oxygen or a group -NH-, Q is hydrogen or a methyl group and Z is a methyl group, a group -OR or -NRR', where R and R', independently of one another, may be hydrogen, a methyl group or an ethyl group, and if R¹⁴ is hydrogen, R¹⁵ can also be C₁₋₄-alkylamino, and R¹⁸ is a straight-chain or branched C₁₋₆-alkyl group, a C₁₋₄-hydroxyalkyl group, a C₁₋₄-aminoalkyl group, an N-C₁₋₄-alkyl-C₁₋₄-aminoalkyl group, an N,N-C₁₋₄-dialkyl-C₁₋₄-aminoalkyl group, an N-C₁₋₄-hydroxyalkyl-C₁₋₄-aminoalkyl group, a C₁₋₄-alkoxymethyl group, a phenyl group, which is optionally substituted by a halogen atom, a C₁₋₄-alkyl group, a C₁₋₄-alkoxy group, a nitro group, a trifluoromethyl group, an amino group or a C₁₋₄-alkylamino group, a benzyl group, which is optionally substituted by a halogen atom, a C₁₋₄-alkyl group, a C₁₋₄-alkoxy group, an amino group, a trifluoromethyl group, a C₁₋₄-alkylamino group or a nitro group, a heterocycle, which is chosen from thiophene, furan and pyridine, or a group -(CH₂)ₚ-O-(CH₂)_{q}-OR", in which R" is hydrogen or a methyl group and p and q are integers which, independently of one another, are in the range from 1 to 3, or is hydrogen with the proviso that R¹³ is then different from an unsubstituted or substituted benzyl group,
and at least one halogenated m-aminophenol of the general formula (I) or one of its physiologically compatible salts as coupler component, 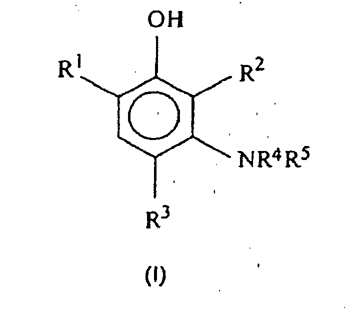 where R¹ is hydrogen or a methyl group,
R² is hydrogen or a halogen atom,
R³ is a chlorine atom,
and R⁴ and R⁵ are both hydrogen,
with the proviso that, if the 4,5-diaminopyrazole derivative is 4,5-diamino-1-hydroxyethylpyrazole and the compound of the formula (I) is 4-chloro-2-methyl-5-aminophenol, it is free from N,N'-bis-(4-aminophenyl)-1,4-diazacycloheptane.

2. Oxidation colorant according to Claim 1, **characterized in that** R² is hydrogen or a chlorine atom.

3. Oxidation colorant according to one of Claims 1 to 2, **characterized in that** the halogenated m-aminophenol is chosen from 2,4-dichloro-3-aminophenol and 4-chloro-2-methyl-5-aminophenol or one of the physiologically compatible salts of these compounds.

4. Oxidation colorant according to one of Claims 1 to 3, **characterized in that** it comprises, as developer component, at least one 4,5-diaminopyrazole derivative chosen from 4,5-diaminopyrazole, 4,5-diamino-1-(2'-hydroxyethyl)pyrazole, 4,5-diamino-1,3-dimethylpyrazole, 4,5-diamino-3-methyl-1-phenylpyrazole, 4,5-diamino-1-methyl-3-phenylpyrazole, 1-benzyl-4,5-diamino-3-methylpyrazole, 4,5-diamino-1-tert-butyl-3-methylpyrazole, 4,5-diamino-1-(2'-hydroxyethyl)-3-methylpyrazole, 4,5-diamino-1-ethyl-3-methylpyrazole, 4,5-diamino-1-ethyl-3-(4'-methoxyphenyl)pyrazole, 4,5-diamino-1-ethyl-3-hydroxymethylpyrazole, 4,5-diamino-3-hydroxymethyl-1-methylpyrazole, 4,5-diamino-3-hydroxymethyl-1-isopropylpyrazole, 4,5-diamino-3-methyl-1-isopropylpyrazole or one of the physiologically compatible salts of these compounds.

5. Oxidation colorant according to Claim 4, **characterized in that** it comprises at least one developer component chosen from 4,5-diaminopyrazole, 4,5-diamino-1-(2'-hydroxyethyl)pyrazole or one of the physiologically compatible salts of these compounds.

6. Oxidation colorant according to one of Claims 1 to 5, **characterized in that** it comprises at least one further developer component.

7. Oxidation colorant according to Claim 6, **characterized in that** a further developer component, chosen from p-phenylenediamine, p-tolylenediamine, p-aminophenol, 1-(2'-hydroxyethyl)-2,5-diaminobenzene, N,N-bis(2'-hydroxyethyl)-p-phenylenediamine, 4-amino-3-methylphenol, 4-amino-2-((diethylamino)methyl)phenol, 2-aminomethyl-4-aminophenol, 2,4,5,6-tetraaminopyrimidine, 2-hydroxy-4,5,6-triaminopyrimidine and 4-hydroxy-2,5,6-triaminopyrimidine, is present.

8. Oxidation colorant according to one of Claims 1 to 7, **characterized in that** it comprises at least one further coupler component.

9. Oxidation colorant according to Claim 8, **characterized in that** it comprises at least one coupler component chosen from 1-naphthol, 1,5-, 2,7- and 1,7-dihydroxynaphthalene, 3-aminophenol, 5-amino-2-methylphenol, 2-chloro-6-methyl-3-aminophenol, 2-amino-3-hydroxypyridine, resorcinol, 4-chlororesorcinol, 2-methylresorcinol, 5-methylresorcinol, 2,5-dimethylresorcinol or 2,6-dihydroxy-3,4-dimethylpyridine.

10. Oxidation colorant according to one of Claims 1 to 9, **characterized in that** developer components are present in an amount of from 0.005 to 20% by weight, preferably 0.1 to 5% by weight, and coupler components are present in an amount of from 0.005 to 20% by weight, preferably 0.1 to 5% by weight, in each case based on the total oxidation colorant.

11. Oxidation colorant according to one of Claims 1 to 10, **characterized in that** at least one direct dye is also present.

12. Method of dyeing keratin fibres, **characterized in that** an oxidation colorant according to one of Claims 1 to 11 is applied, in a suitable cosmetic preparation, to the fibres to be dyed and the colour is developed with the help of an oxidizing agent **in that** the oxidizing agent is either added to the colorant directly prior to use and then applied to the fibres, or else is applied to the fibres to be dyed at the same time as the colorant or directly after the colorant, then the entire preparation remains on the fibres for about 10 to 45 minutes, preferably 30 minutes, and is then rinsed out again or washed out with a shampoo.

## Revendications

1. Teinture d'oxydation pour la teinture de fibres kératiniques, **caractérisée en ce qu'**elle contient une combinaison d'au moins un dérivé du 4,5-diaminopyrazole répondant à la formule générale (II) ou d'un de ses sels physiologiquement acceptables 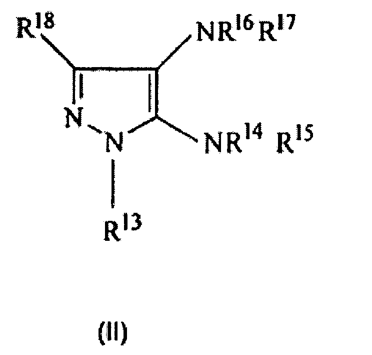 dans laquelle R¹³, R¹⁴, R¹⁵, R¹⁶ et R¹⁷ représentent, indépendamment l'un de l'autre, un atome d'hydrogène, un groupe alkyle en C₁-C₆ à chaîne droite ou ramifiée, un groupe hydroxyalkyle en C₂-C₄, un groupe aminoalkyle en C₂-C₄, un groupe phényle qui, le cas échéant, est substitué avec un groupe nitro, un groupe trifluorométhyle, un groupe amino ou un groupe alkyl(en C₁-C₄)amino, un groupe benzyle qui, le cas échéant, est substitué avec un atome d'halogène, un groupe alkyle en C₁₋C₄, un groupe alcoxy en C₁-C₄, un groupe méthylènedioxy ou un groupe amino, un groupe pyridyle, un groupe thiényle, un groupe furyle ou un groupe 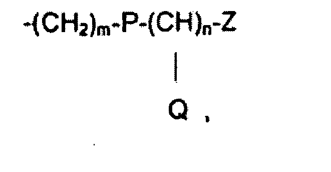 dans lequel m et n représentent, indépendamment l'un de l'autre, des nombres entiers dans la plage de 1 à 3, P représente un atome d'oxygène ou un groupe ―NH-, Q représente un atome d'hydrogène ou un groupe méthyle et Z représente un groupe méthyle, un groupe ―OR ou un groupe ―NRR', R et R' pouvant représenter, indépendamment l'un de l'autre, un atome d'hydrogène, un groupe méthyle ou un groupe éthyle, et, dans le cas où R¹⁴ représente un atome d'hydrogène, R¹⁵ peut également représenter un groupe alkyl(en C₁-C₄)amino,
et R¹⁸ représente un groupe alkyle en C₁-C₆ à chaîne droite ou ramifiée, un groupe hydroxyalkyle en C₁-C₄, un groupe aminoalkyle en C₁-C₄, un groupe N-alkyl(en C₁-C₄)aminoalkyle en C₁-C₄, un groupe N,N-dialkyl(en C₁-C₄)aminoalkyle en C₁-C₄, un groupe N-hydroxy-alkyl(en C₁₋C₄)aminoalkyle en C₁-C₄, un groupe alcoxyméthyle en C₁-C₄, un groupe phényle qui, le cas échéant, est substitué avec un atome d'halogène, un groupe alkyle en C₁-C₄, un groupe alcoxy en C₁-C₄, un groupe nitro, un groupe trifluorométhyle, un groupe amino ou un groupe alkyl(en C₁₋C₄)amino, un groupe benzyle qui, le cas échéant, est substitué avec un atome d'halogène, un groupe alkyle en C₁-C₄, un groupe alcoxy en C₁-C₄, un groupe amino, un groupe trifluorométhyle, un groupe alkyl(en C₁₋C₄)amino ou un groupe nitro, un composé hétérocyclique qui est choisi parmi le groupe comprenant le thiophène, le furane et la pyridine, ou un groupe ―(CH₂)ₚ-O-(CH₂)_{q}-OR" où R" représente un atome d'hydrogène ou un groupe méthyle, et p et q représentent des nombres entiers qui, indépendamment l'un de l'autre, se situent dans la plage de 1 à 3, ou un atome d'hydrogène avec cette mesure que R¹³ est alors différent d'un groupe benzyle non substitué ou substitué,
et d'au moins un m-aminophénol halogéné répondant à la formule générale (I) ou d'un de ses sels physiologiquement acceptables, à titre de composant faisant office de coupleur, 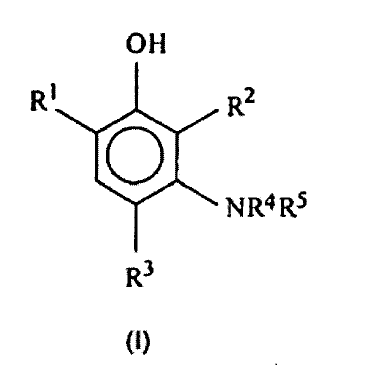 dans laquelle R¹ représente un atome d'hydrogène ou un groupe méthyle ;
R² représente un atome d'hydrogène ou un atome d'halogène ;
R³ représente un atome de chlore,
et R⁴ et R⁵ représentent tous deux un atome d'hydrogène ;
avec cette mesure que, lorsque le dérivé de 4,5-diaminopyrazole est le 4,5-diamino-1-hydroxyéthylpyrazole et lorsque le composé répondant à la formule I est le 4-chloro-2-méthyl-5-aminophénol, elle est exempte du N,N'-bis(4-aminophényl)-1,4-diazacycloheptane.

2. Teinture d'oxydation selon la revendication 1, **caractérisée en ce que** R² représente un atome d'hydrogène ou un atome de chlore.

3. Teinture d'oxydation selon l'une quelconque des revendications 1 ou 2, **caractérisée en ce que** le m-aminophénol halogéné est choisi parmi le groupe constitué par le 2,4-dichloro-3-aminophénol et par le 4-chloro-2-méthyl-5-aminophénol ou représente un des sels physiologiquement acceptables de ces composés.

4. Teinture d'oxydation selon l'une quelconque des revendications 1 à 3, **caractérisée en ce qu'**elle contient, à titre de composant faisant office de développeur, au moins un dérivé du 4,5-diaminopyrazole, choisi parmi le groupe comprenant le 4,5-diaminopyrazole, le 4,5-diamino-1-(2'-hydroxyéthyl)-pyrazole, le 4,5-diamino-1,3-diméthyl-pyrazole, le 4,5-diamino-3-méthyl-1-phényl-pyrazole, le 4,5-diamino-1-méthyl-3-phényl-pyrazole, le 1-benzyl-4,5-diamino-3-méthyl-pyrazole, le 4,5-diamino-1-tert-butyl-3-méthyl-pyrazole, le 4,5-diamino-1-(2'-hydroxyéthyl)-3-méthyl-pyrazole, le 4,5-diamino-1-éthyl-3-méthyl-pyrazole, le 4,5-diamino-1-éthyl-3-(4'-méthoxyphényl)-pyrazole, le 4,5-diamino-1-éthyl-3-hydroxyméthyl-pyrazole, le 4,5-diamino-3-hydroxyméthyl-1-méthyl-pyrazole, le 4,5-diamino-3-hydroxyméthyl-1-isopropyl-pyrazole, le 4,5-diamino-3-méthyl-1-isopropyl-pyrazole ou un des sels physiologiquement acceptables de ces composés.

5. Teinture d'oxydation selon la revendication 4, **caractérisée en ce qu'**elle contient au moins un composant faisant office de développeur choisi parmi le groupe comprenant le 4,5-diaminopyrazole, le 4,5-diamino-1-(2'-hydroxyéthyl)-pyrazole ou un des sels physiologiquement acceptables de ces composés.

6. Teinture d'oxydation selon l'une quelconque des revendications 1 à 5, **caractérisée en ce qu'**elle contient au moins un composant supplémentaire faisant office de développeur.

7. Teinture d'oxydation selon la revendication 6, **caractérisée en ce qu'**elle contient un composant supplémentaire faisant office de développeur choisi parmi le groupe comprenant la p-phénylènediamine, la p-toluylènediamine, le p-aminophénol, le 1-(2'-hydroxyéthyl)-2,5-diaminobenzène, la N,N-bis-(2'-hydroxy-éthyl)-p-phénylènediamine, le 4-amino-3-méthylphénol, le 4-amino-2-((diéthylamino)-méthyl)-phénol, le 2-aminométhyl-4-aminophénol, la 2,4,5,6-tétra-aminopyrimidine, la 2-hydroxy-4,5,6-triaminopyrimidine et la 4-hydroxy-2,5,6-triaminopyrimidine.

8. Teinture d'oxydation selon l'une quelconque des revendications 1 à 7, **caractérisée en ce qu'**elle contient au moins un composant supplémentaire faisant office de coupleur.

9. Teinture d'oxydation selon la revendication 8, **caractérisée en ce qu'**elle contient au moins un composant faisant office de coupleur choisi parmi le groupe comprenant le 1-naphtol, le 1,5-, le 2,7- et le 1,7-dihydroxynaphtalène, le 3-aminophénol, le 5-amino-2-méthylphénol, le 2-chloro-6-méthyl-3-aminophénol, la 2-amino-3-hydroxypyridine, le résorcinol, le 4-chlororésorcinol, le 2-méthylrésorcinol, le 5-méthylrésorcinol, le 2,5-diméthylrésorcinol ou la 2,6-dihydroxy-3,4-diméthylpyridine.

10. Teinture d'oxydation selon l'une quelconque des revendications 1 à 9, **caractérisée en ce qu'**elle contient des composants faisant office de développeurs en une quantité de 0,005 à 20 % en poids, de préférence de 0,1 à 5 % en poids, et des composants faisant office de coupleurs en une quantité de 0,005 à 20 % en poids, de préférence de 0,1 à 5 % en poids, chaque fois rapportés à la teinture d'oxydation totale.

11. Teinture d'oxydation selon l'une quelconque des revendications 1 à 10, **caractérisée en ce qu'**elle contient en outre un colorant montant directement sur la fibre.

12. Procédé pour la teinture de fibres kératiniques, **caractérisé en ce qu'**on applique une teinture d'oxydation selon l'une quelconque des revendications 1 à 11 dans une préparation cosmétique appropriée sur les fibres à teinter, la teinture étant développée à l'aide d'un agent d'oxydation, soit en ajoutant l'agent d'oxydation à la teinture directement avant l'utilisation et en l'appliquant ensuite sur les fibres, soit en l'appliquant simultanément avec la teinture, ou encore en l'appliquant directement après la teinture sur les fibres à teinter, en laissant ensuite la préparation totale sur les fibres pendant un laps de temps d'environ 10 à 45 minutes, de préférence pendant 30 minutes, avant de l'éliminer par rinçage ou par lavage avec un shampooing.
